# EUROPEAN PATENT APPLICATION

(11) **EP 4 751 736 A1**
(43) Date of publication of application: **03.06.2026**
(21) Application number: 24845685.7
(22) Date of filing: 25.07.2024
(51) Int. Cl.: A61K 47/18, A61K 39/39, A61P 31/00

(54) **COMPOSITION FOR USE IN VACCINE**

(30) Priority: 25.07.2023 US 202363515413 P
(71) Applicant: The University of Osaka, Suita-shi, Osaka 565-0871 (JP); Tohoku University, Sendai-shi, Miyagi 980-8577 (JP); The Research Foundation for Microbial Diseases of Osaka University, Suita-shi, Osaka 565-0871 (JP)
(72) Inventor: YOSHIOKA, Yasuo, Suita-shi, Osaka 565-0871 (JP); AKITA, Hidetaka, Sendai-shi, Miyagi 980-8577 (JP); TANAKA, Hiroki, Sendai-shi, Miyagi 980-8577 (JP); WATANABE, Tokiko, Suita-shi, Osaka 565-0871 (JP); SHIMIZU, Taro, Suita-shi, Osaka 565-0871 (JP)
(74) Representative: Mewburn Ellis LLP
(86) International application number: PCT/JP2024/026712
(87) International publication number: WO 2025/023311

(57) **Abstract**

The present invention relates to a composition for use in a vaccine, the composition comprising a cationic lipid represented by Formula (1) below.

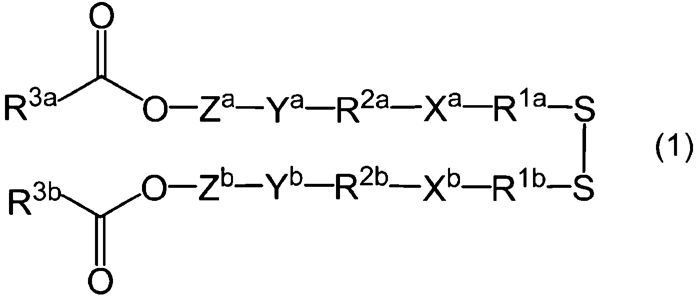

## Description

### Technical Field

The present invention relates to a composition for use in a vaccine.

### Background Art

Vaccines (mRNA-LNP vaccines) composed of lipid nanoparticles (hereinafter, referred to as "LNP") encapsulating messenger RNA (mRNA) are expected to be a highly effective modality, as these vaccines can be suitably adopted for the 100 Days Mission. While the current mRNA-LNP vaccines strongly induce vaccine effects, adverse reactions caused by the production of inflammatory cytokines such as fever, swelling at the administration site, and malaise are frequently observed, which may trigger aversion to the vaccines. Therefore, to elevate the vaccines to a modality that can be applied not only in emergency situations but also in ordinary times, it is essential to reduce the adverse reactions caused by the prophlogistic properties while maintaining the vaccine effects.

In addition, although cationic lipids exhibiting improved intracellular dynamics are disclosed in Patent Literature 1, the use of the cationic lipids in vaccines is not disclosed therein.

### Citation List

### Patent Literature

Patent Literature 1: WO 2019/188867

### Summary of Invention

### Technical Problem

With the foregoing problems in view, it is an object of the present invention to provide a composition for use in a vaccine, the composition being capable of reducing an adverse reaction.

### Solution to Problem

As a result of extensive research, the present inventors discovered a composition for use in a vaccine, the composition being capable of reducing an adverse reaction, and completed the present invention.

That is, the present invention comprises the following aspects.
[1] A composition for use in a vaccine, the composition comprising a cationic lipid represented by the following Formula (1): 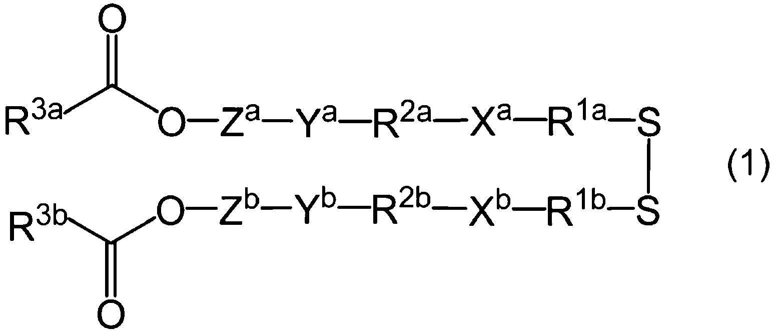 (In Formula (1),
   R^{1a} and R^{1b} each independently represent an alkylene group having 1 to 6 carbon atoms,
   X^{a} and X^{b} each independently represent an acyclic alkyl tertiary amino group having one tertiary amino group wherein the alkyl has 1 to 6 carbon atoms or a cyclic alkylene tertiary amino group having one or two tertiary amino groups wherein the alkylene has 2 to 5 carbon atoms,
   R^{2a} and R^{2b} each independently represent an alkylene or an oxydialkylene group having 8 or less carbon atoms,
   Y^{a} and Y^{b} each independently represent an ester bond, an amide bond, a carbamate bond, an ether bond, or a urea bond,
   Z^{a} and Z^{b} each independently represent a divalent group derived from an aromatic compound, the aromatic compound having 3 to 16 carbon atoms and at least one aromatic ring and optionally having a heteroatom, and
   R^{3a} and R^{3b} each independently represent a residue derived from a reaction product of a fat-soluble vitamin having a hydroxyl group and succinic anhydride or glutaric anhydride, a residue derived from a reaction product of a sterol derivative having a hydroxyl group and succinic anhydride or glutaric anhydride, or an aliphatic hydrocarbon group having 12 to 22 carbon atoms).
[2] The composition according to [1], further comprising a nucleic acid.
[3] The composition according to [2], wherein a ratio of a content of the cationic lipid and a content of the nucleic acid, as a content of cationic lipid (nmol)/content of nucleic acid (µg), is 0.05 to 150.
[4] The composition according to any of [1] to [3], further comprising one or more selected from the group consisting of an ionizable lipid, a neutral lipid, a PEG-modified lipid, and a sterol.
[5] The composition according to [1] to [4], wherein the composition forms lipid particles.
[6] The composition according to any of [1] to [5], which is for preventing or treating an infectious disease.
[7] The composition according to any of [1] to [6], which is for intradermal administration, subcutaneous administration, transnasal administration, or intramuscular administration.
[8] The composition according to [1] to [7], in which the prevention of the infectious disease comprises inducing protective immunity against an infectious disease in a subject.

The present invention also comprises following aspects.
[A1] A vaccine comprising a cationic lipid represented by Formula (1) above.
[A2] The vaccine according to [A1], further comprising a nucleic acid.
[A3] The vaccine according [A1] or [A2], wherein a ratio of a content of the cationic lipid and a content of the nucleic acid, as a content of cationic lipid (nmol)/content of nucleic acid (µg), is 0.05 to 150.
[A4] The vaccine according to any of [A1] to [A3], further comprising one or more selected from the group consisting of an ionizable lipid, a neutral lipid, a PEG-modified lipid, and a sterol.
[A5] The composition according to [A1] to [A4], which is lipid particles.
[A6] The vaccine according to any of [A1] to [A5], which is for preventing or treating an infectious disease.
[A7] The vaccine according to any of [A1] to [A6], which is for intradermal administration, subcutaneous administration, transnasal administration, or intramuscular administration.
[A8] The vaccine according to [A1] to [A7], in which the prevention of the infectious disease comprises inducing protective immunity against an infectious disease in a subject.
[B1] A method for preventing a disease comprising administering a composition comprising a cationic lipid represented by Formula (1) above and a nucleic acid.
[B2] The method according to [B1], in which a ratio of a content of the cationic lipid and a content of the nucleic acid, as a content of cationic lipid (nmol)/content of nucleic acid (µg), is 0.05 to 150.
[B3] The method according to [B1] or [B2], in which the composition further comprises one or more selected from the group consisting of an ionizable lipid, a neutral lipid, a PEG-modified lipid, and a sterol.
[B4] The composition according to [B1] to [B3], which is lipid particles.
[B5] The method according to any of [B1] to [B4], in which the disease is an infectious disease.
[B6] The method according to any of [B1] to [B5], in which the composition is administered by intradermal administration, subcutaneous administration, transnasal administration, or intramuscular administration.
[B7] The method according to any of [B1] to [B6], in which the composition induces protective immunity against a disease in a subject.
[C1] A method for inducing protective immunity against a disease in a subject, comprising administering a composition represented by Formula (1) above, the composition comprising a cationic lipid and a nucleic acid.
[C2] The method according to [C1], in which a ratio of a content of the cationic lipid and a content of the nucleic acid, as a content of cationic lipid (nmol)/content of nucleic acid (µg). is 0.05 to 150.
[C3] The method according to [C1] or [C2], wherein the composition comprises one or more selected from the group consisting of an ionizable lipid, a neutral lipid, a PEG-modified lipid, and a sterol.
[C4] The composition according to [C1] to [C3], which is lipid particles.
[C5] The method according to any of [C1] to [C4], in which the disease is an infectious disease.
[C6] The method according to any of [C1] to [C5], in which the composition is administered by intradermal administration, subcutaneous administration, transnasal administration, or intramuscular administration.
[D1] Use of a composition for preventing a disease, the composition comprising a cationic lipid represented by Formula (1) above and a nucleic acid.
[D2] The use according to [D1], in which a ratio of a content of the cationic lipid and a content of the nucleic acid, as a content of cationic lipid (nmol)/content of nucleic acid (µg), is 0.05 to 150.
[D3] The use according to [D1] or [D2], in which the composition further comprises one or more selected from the group consisting of an ionizable lipid, a neutral lipid, a PEG-modified lipid, and a sterol.
[D4] The composition according to [D1] to [D3], which is lipid particles.
[D5] The use according to any of [D1] to [D4], in which the disease is an infectious disease.
[D6] The use according to any of [D1] to [D5], in which the composition is administered by intradermal administration, subcutaneous administration, transnasal administration, or intramuscular administration.
[D7] The use according to any of [D1] to [D6], in which the prevention of the infectious disease comprises inducing protective immunity against an infectious disease in a subject.
[E1] Use of a cationic lipid represented by Formula (1) above in production of a composition for use in a vaccine.
[E2] The use according to [E1], in which the composition further comprises a nucleic acid.
[E3] The use according to [E2], in which a ratio of a content of the cationic lipid and a content of the nucleic acid, as a content of cationic lipid (nmol)/content of nucleic acid (µg), is 0.05 to 150.
[E4] The use according to any of [E1] to [E3], in which the composition further comprises one or more selected from the group consisting of an ionizable lipid, a neutral lipid, a PEG-modified lipid, and a sterol.
[E5] The composition according to any of [E1] to [E4], which is lipid particles.
[E6] The composition according to any of [E1] to [E5], which is for preventing or treating an infectious disease.
[E7] The use according to any of [E1] to [E6], which is for administering the composition by intradermal administration, subcutaneous administration, transnasal administration, or intramuscular administration.
[E8] The use according to [E1] to [E7], in which the prevention of the infectious disease comprises inducing protective immunity against an infectious disease in a subject.

The use in [E1] to [E8] may be use of the cationic lipid represented by Formula (1) above in production of a vaccine.

### Advantageous Effect of Invention

According to the present invention, a composition for use in a vaccine can be provided, the composition being capable of reducing an adverse reaction.

### Brief Description of Drawings

[Figure 1] Figure 1 is a diagram showing the results of measuring an encapsulation rate of mRNA-LNPs.
[Figure 2] Figure 2 shows cryo-electron microscope images of mRNA-LNPs.
[Figure 3] Figure 3 is a diagram showing antibody titers after two subcutaneous immunizations.
[Figure 4] Figure 4 is a diagram showing antibody titers after a subcutaneous prime immunization.
[Figure 5] Figure 5 is a diagram comparing antibody titers after two subcutaneous immunizations and antibody titers of a control.
[Figure 6] Figure 6 is a diagram showing antibody titers after two intramuscular immunizations.
[Figure 7] Figure 7 is a diagram showing T cell responses after two subcutaneous immunizations or after two intramuscular immunizations.
[Figure 8] Figure 8 is a diagram showing the ability to protect against an infection using the body weight and survival rate as indicators.
[Figure 9] Figure 9 is a diagram showing mRNA transfection efficiency after one intramuscular immunization.
[Figure 10] Figure 10 is a diagram showing mRNA transfection efficiency after two intramuscular immunizations.
[Figure 11] Figure 11 is a diagram showing HA expressions after two intramuscular immunizations.
[Figure 12] Figure 12 is a diagram showing activation of antigen-presenting cells in draining lymph nodes after a subcutaneous immunization.
[Figure 13] Figure 13 is a diagram showing blood cytokine levels after subcutaneous immunizations.
[Figure 14] Figure 14 is a diagram showing blood cytokine levels after subcutaneous immunizations.
[Figure 15] Figure 15 is a diagram showing blood cytokine levels after intramuscular immunizations.
[Figure 16] Figure 16 is a diagram showing adverse reactions after an intramuscular immunization.
[Figure 17] Figure 17 is a diagram comparing blood cytokine levels and antibody titers between LNPs using different neutral lipids.

### Description of Embodiments

Hereinafter, the embodiments of the present invention will be described in detail. The present invention is not limited to the following embodiments, and various changes can be made within the scope of the gist thereof.

A composition of the present embodiment comprises a cationic lipid represented by the following Formula (1) below. 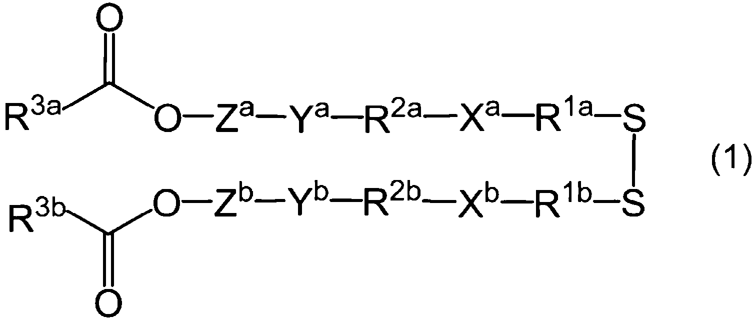 (In Formula (1),
R^{1a} and R^{1b} each independently represent an alkylene group having 1 to 6 carbon atoms,
X^{a} and X^{b} each independently represent an acyclic alkyl tertiary amino group having one tertiary amino group wherein the alkyl has 1 to 6 carbon atoms or a cyclic alkylene tertiary amino group having one or two tertiary amino groups wherein the alkylene has 2 to 5 carbon atoms,
R^{2a} and R^{2b} each independently represent an alkylene or an oxydialkylene group having 8 or less carbon atoms,
Y^{a} and Y^{b} each independently represent an ester bond, an amide bond, a carbamate bond, an ether bond, or a urea bond,
Z^{a} and Z^{b} each independently represent a divalent group derived from an aromatic compound, the aromatic compound having 3 to 16 carbon atoms and at least one aromatic ring and optionally having a heteroatom, and
R^{3a} and R^{3b} each independently represent a residue derived from a reaction product of a fat-soluble vitamin having a hydroxyl group and succinic anhydride or glutaric anhydride, a residue derived from a reaction product of a sterol derivative having a hydroxyl group and succinic anhydride or glutaric anhydride, or an aliphatic hydrocarbon group having 12 to 22 carbon atoms)

In the present embodiment, R^{1a} and R^{1b} each independently represent an alkylene group having 1 to 6 carbon atoms, which may be linear or branched. Although the structure is not particularly limited, R^{1a} and R^{1b} are preferably linear. Although the number of carbon atoms in the alkylene group is not particularly limited, the alkylene group preferably has, for example, 1 to 4 carbon atoms, and more preferably has 1 or 2 carbon atoms.

The alkylene group having 1 to 6 carbon atoms is not particularly limited, and examples thereof include a methylene group, an ethylene group, a trimethylene group, an isopropylene group, a tetramethylene group, an isobutylene group, a pentamethylene group, and a neopentylene group. Among these groups, the alkylene group is preferably a methylene group, an ethylene group, a trimethylene group, an isopropylene group, or a tetramethylene group, and more preferably an ethylene group.

R^{1a} may be the same as or different from R^{1b}, and although there are no particular limitations, it is preferable that R^{1a} is the same group as R^{1b}.

In the present embodiment, X^{a} and X^{b} each independently represent an acyclic alkyl tertiary amino group having one tertiary amino group wherein the alkyl has 1 to 6 carbon atoms or a cyclic alkylene tertiary amino group having one or two tertiary amino groups wherein the alkylene has 2 to 5 carbon atoms. Although X^{a} and X^{b} are not particularly limited, it is preferable that X^{a} and X^{b} are each independently a cyclic alkylene tertiary amino group having one or two tertiary amino groups wherein the alkylene has 2 to 5 carbon atoms.

X^{a} may be the same as or different from X^{b}, and although there are no particular limitations, it is preferable that X^{a} is the same group as X^{b}.

In the acyclic alkyl tertiary amino group having one tertiary amino group wherein the alkyl has 1 to 6 carbon atoms, the alkyl group having 1 to 6 carbon atoms may be linear, branched, or cyclic. Although the number of carbon atoms in the alkyl group is not particularly limited, the alkyl group preferably has, for example, 1 to 3 carbon atoms.

The alkyl group having 1 to 6 carbon atoms is not particularly limited, and examples thereof comprise a methyl group, an ethyl group, a propyl group, an isopropyl group, a n-butyl group, a sec-butyl group, an isobutyl group, a tert-butyl group, a pentyl group, an isopentyl group, a neopentyl group, a t-pentyl group, a 1,2-dimethylpropyl group, a 2-methylbutyl group, a 2-methylpentyl group, a 3-methylpentyl group, a 2,2-dimethylbutyl group, a 2,3-dimethylbutyl group, and a cyclohexyl group. Among these groups, the alkyl group is preferably a methyl group, an ethyl group, a propyl group, or an isopropyl group, and more preferably a methyl group.

A specific structure of the acyclic alkyl tertiary amino group having one tertiary amino group wherein the alkyl has 1 to 6 carbon atoms is preferably represented by X¹.

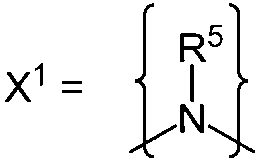

R⁵ of X¹ represents an alkyl group having 1 to 6 carbon atoms, which may be linear, branched, or cyclic. Although the number of carbon atoms in the alkyl group is not particularly limited, the alkyl group preferably has, for example, 1 to 3 carbon atoms. The alkyl group having 1 to 6 carbon atoms is not particularly limited, and examples thereof comprise a methyl group, an ethyl group, a propyl group, an isopropyl group, a n-butyl group, a sec-butyl group, an isobutyl group, a tert-butyl group, a pentyl group, an isopentyl group, a neopentyl group, a t-pentyl group, a 1,2-dimethylpropyl group, a 2-methylbutyl group, a 2-methylpentyl group, a 3-methylpentyl group, a 2,2-dimethylbutyl group, a 2,3-dimethylbutyl group, and a cyclohexyl group. Among these groups, the alkyl group is preferably a methyl group, an ethyl group, a propyl group, or an isopropyl group, and more preferably a methyl group.

Although the number of carbon atoms in the cyclic alkylene tertiary amino group having one or two tertiary amino groups wherein the alkylene has 2 to 5 carbon atoms is not particularly limited, the alkylene in the cyclic alkylene tertiary amino group preferably has, for example, 4 or 5 carbon atoms.

Although the cyclic alkylene tertiary amino group having one or two tertiary amino groups wherein the alkylene has 2 to 5 carbon atoms is not particularly limited, examples thereof comprise an aziridylene group, an azetidylene group, a pyrrolidylene group, a piperidylene group, an imidazolidylene group, and a piperazylene group. Among these groups, the cyclic alkylene tertiary amino group is preferably a pyrrolidylene group, a piperidylene group, or a piperazylene group, and more preferably a piperidylene group.

A specific structure of the cyclic alkylene tertiary amino group having one tertiary amino group wherein the alkylene has 2 to 5 carbon atoms is preferably represented by X². 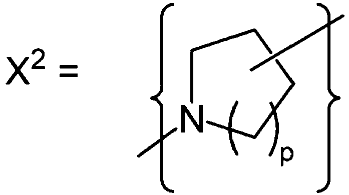 p in X² is 1 or 2. When p is 1, X² is a pyrrolidylene group, and when p is 2, X² is a piperidylene group.

A specific structure of the cyclic alkylene tertiary amino group having two tertiary amino groups wherein the alkylene has 2 to 5 carbon atoms is preferably represented by X³. 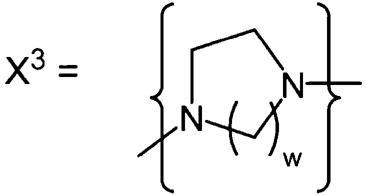 w in X³ is 1 or 2. When w is 1, X³ is an imidazolidylene group, and when w is 2, X³ is a piperazylene group.

In the present embodiment, R^{2a} and R^{2b} each independently represent an alkylene or an oxydialkylene group having 8 or less carbon atoms. Although R^{2a} and R^{2b} are not particularly limited, it is preferable that R^{2a} and R^{2b} are each independently an alkylene group having 8 or less carbon atoms.

R^{2a} may be the same as or different from R^{2b}, and although there are no particular limitations, it is preferable that R^{2a} is the same group as R^{2b}.

The alkylene group having 8 or less carbon atoms may be linear or branched, and although the structure of the alkylene group is not particularly limited, the alkylene group is preferably linear. Although the number of carbon atoms in the alkylene group is not particularly limited, the alkylene group preferably has 6 or less carbon atoms, and more preferably has 4 or less carbon atoms. The alkylene group having 8 or less carbon atoms is not particularly limited, and examples thereof comprise a methylene group, an ethylene group, a propylene group, an isopropylene group, a tetramethylene group, an isobutylene group, a pentamethylene group, a hexamethylene group, a heptamethylene group, and an octamethylene group. Among these groups, the alkylene group is preferably a methylene group, an ethylene group, a propylene group, or a tetramethylene group, and more preferably an ethylene group.

The oxydialkylene group having 8 or less carbon atoms refers to alkylene groups linked via an ether bond (alkylene-O-alkylene), and the total number of carbon atoms in the two alkylene groups is 8 or less. Here, the two alkylenes may be the same as or different from each other, but are preferably the same. The oxydialkylene group having 8 or less carbon atoms is not particularly limited, and examples thereof comprise an oxydimethylene group, an oxydiethylene group, an oxydipropylene group, and an oxydibutylene group. Among these groups, the oxydialkylene group is preferably an oxydimethylene group, an oxydiethylene group, or an oxydipropylene group, and more preferably an oxydiethylene group.

In the present embodiment, Y^{a} and Y^{b} each independently represent an ester bond, an amide bond, a carbamate bond, an ether bond, or a urea bond. Although Y^{a} and Y^{b} are not particularly limited, it is preferable that Y^{a} and Y^{b} are each independently an ester bond, an amide bond, or a carbamate bond, and it is more preferable that Y^{a} and Y^{b} are each independently an ester bond or an amide bond, and still more preferably each independently an ester bond. The directions of the bonds Y^{a} and Y^{b} are not particularly limited, but when Y^{a} and Y^{b} are ester groups, the structures -Z^{a}-CO-O-R^{2a}- and -Z^{b}-CO-O-R^{2b}- are preferable.

Y^{a} may be the same as or different from Y^{b}, and although there are no particular limitations, it is preferable that Y^{a} is the same group as Y^{b}.

In the present embodiment, Z^{a} and Z^{b} each independently represent a divalent group derived from an aromatic compound which may have a heteroatom, the aromatic compound having 3 to 16 carbon atoms and at least one aromatic ring. The number of carbon atoms in the aromatic compound is preferably 6 to 12, and more preferably 6 or 7. Also, it is preferable that the aromatic compound comprises one aromatic ring.

The aromatic ring in the aromatic compound having 3 to 16 carbon atoms is not particularly limited, and examples thereof comprise an aromatic hydrocarbon ring and an aromatic heterocycle. The aromatic hydrocarbon ring is not particularly limited, and examples thereof comprise a benzene ring, a naphthalene ring, and an anthracene ring. The aromatic heterocycle is not particularly limited, and examples thereof comprise an imidazole ring, a pyrazole ring, an oxazole ring, an isoxazole ring, a thiazole ring, an isothiazole ring, a triazine ring, a pyrrole ring, a furanthiophene ring, a pyrimidine ring, a pyridazine ring, a pyrazine ring, a pyridine ring, a purine ring, a pteridine ring, a benzimidazole ring, an indole ring, a benzofuran ring, a quinazoline ring, a phthalazine ring, a quinoline ring, an isoquinoline ring, a coumarin ring, a chromone ring, a benzodiazepine ring, a phenoxazine ring, a phenothiazine ring, and an acridine ring. Among these, the aromatic ring is preferably a benzene ring, a naphthalene ring, or an anthracene ring, and more preferably a benzene ring.

The aromatic ring may have a substituent. The substituent is not particularly limited, and examples thereof comprise an acyl group having 2 to 4 carbon atoms, an alkoxycarbonyl group having 2 to 4 carbon atoms, a carbamoyl group having 2 to 4 carbon atoms, an acyloxy group having 2 to 4 carbon atoms, an acylamino group having 2 to 4 carbon atoms, an alkoxycarbonylamino group having 2 to 4 carbon atoms, a fluorine atom, a chlorine atom, a bromine atom, an iodine atom, an alkylsulfanyl group having 1 to 4 carbon atoms, an alkylsulfonyl group having 1 to 4 carbon atoms, an arylsulfonyl group having 6 to 10 carbon atoms, a nitro group, a trifluoromethyl group, a cyano group, an alkyl group having 1 to 4 carbon atoms, a ureido group having 1 to 4 carbon atoms, an alkoxy group having 1 to 4 carbon atoms, an aryl group having 6 to 10 carbon atoms, and an aryloxy group having 6 to 10 carbon atoms. Among these, the substituent is preferably an acetyl group, a methoxycarbonyl group, a methylcarbamoyl group, an acetoxy group, an acetamide group, a methoxycarbonylamino group, a fluorine atom, a chlorine atom, a bromine atom, an iodine atom, a methylsulfanyl group, a phenylsulfonyl group, a nitro group, a trifluoromethyl group, a cyano group, a methyl group, an ethyl group, a propyl group, an isopropyl group, a t-butyl group, a ureido group, a methoxy group, an ethoxy group, a propoxy group, an isopropoxy group, a t-butoxy group, a phenyl group, a phenoxy group, or the like.

Z^{a} may be the same as or different from Z^{b}, and although there are no particular limitations, it is preferable that Z^{a} is the same group as Z^{b}.

Specific structures of Z^{a} and Z^{b} are preferably represented by Z¹.

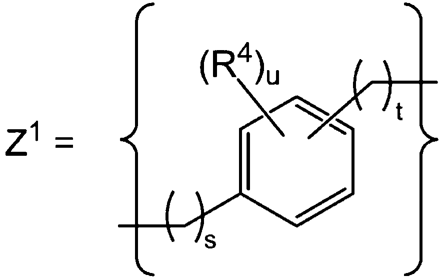

In Z¹, s represents an integer of 0 to 3, t represents an integer of 0 to 3, u represents an integer of 0 to 4, and R^{4'}s, of which the number is u, each independently represent a substituent.

s in Z¹ is preferably an integer of 0 or 1, and more preferably 0.

t in Z¹ is preferably an integer of 0 to 2, and more preferably 1.

u in Z¹ is preferably an integer of 0 to 2, and more preferably an integer of 0 or 1.

R⁴ in Z¹ is a substituent of the aromatic ring (benzene ring) in the aromatic compound having 3 to 16 carbon atoms that does not inhibit the reaction in the synthesis process of the cationic lipid represented by Formula (1) above. The substituent is not particularly limited, and examples thereof comprise an acyl group having 2 to 4 carbon atoms, an alkoxycarbonyl group having 2 to 4 carbon atoms, a carbamoyl group having 2 to 4 carbon atoms, an acyloxy group having 2 to 4 carbon atoms, an acylamino group having 2 to 4 carbon atoms, an alkoxycarbonylamino group having 2 to 4 carbon atoms, a fluorine atom, a chlorine atom, a bromine atom, an iodine atom, an alkylsulfanyl group having 1 to 4 carbon atoms, an alkylsulfonyl group having 1 to 4 carbon atoms, an arylsulfonyl group having 6 to 10 carbon atoms, a nitro group, a trifluoromethyl group, a cyano group, an alkyl group having 1 to 4 carbon atoms, a ureido group having 1 to 4 carbon atoms, an alkoxy group having 1 to 4 carbon atoms, an aryl group having 6 to 10 carbon atoms, and an aryloxy group having 6 to 10 carbon atoms. Among these, the substituent is preferably an acetyl group, a methoxycarbonyl group, a methylcarbamoyl group, an acetoxy group, an acetamide group, a methoxycarbonylamino group, a fluorine atom, a chlorine atom, a bromine atom, an iodine atom, a methylsulfanyl group, a phenylsulfonyl group, a nitro group, a trifluoromethyl group, a cyano group, a methyl group, an ethyl group, a propyl group, an isopropyl group, a t-butyl group, a ureido group, a methoxy group, an ethoxy group, a propoxy group, an isopropoxy group, a t-butoxy group, a phenyl group, or a phenoxy group. In a case where a plurality of R^{4'}s are present, R^{4'}s may be the same as or different from each other.

In the present embodiment, R^{3a} and R^{3b} each independently represent a residue derived from a reaction product of a fat-soluble vitamin having a hydroxyl group and succinic anhydride or glutaric anhydride, a residue derived from a reaction product of a sterol derivative having a hydroxyl group and succinic anhydride or glutaric anhydride, or an aliphatic hydrocarbon group having 12 to 22 carbon atoms. Although R^{3a} and R^{3b} are not particularly limited, it is preferable that R^{3a} and R^{3b} are each independently a residue derived from a reaction product of a fat-soluble vitamin having a hydroxyl group and succinic anhydride or glutaric anhydride or an aliphatic hydrocarbon group having 12 to 22 carbon atoms, and it is more preferable that R^{3a} and R^{3b} are each independently an aliphatic hydrocarbon group having 12 to 22 carbon atoms.

When R^{3a} and/or R^{3b} is a residue derived from a reaction product with succinic anhydride or glutaric anhydride, the carbonyl groups which are substituents bonded to R^{3a} and R^{3b} in Formula (1) may be derived from succinic anhydride or glutaric anhydride.

The fat-soluble vitamin having a hydroxyl group is not particularly limited, and examples thereof comprise retinol, ergosterol, 7-dehydrocholesterol, calciferol, colcalciferol, dihydroergocalciferol, dihydrotachysterol, tocopherol, and tocotrienol. Among these, the fat-soluble vitamin is preferably tocopherol.

The sterol derivative having a hydroxyl group is not particularly limited, and examples thereof comprise cholesterol, cholestanol, stigmasterol, β-sitosterol, lanosterol, and ergosterol. Among these, the sterol derivative is cholesterol or cholestanol.

The aliphatic hydrocarbon group having 12 to 22 carbon atoms may be linear or branched. The aliphatic hydrocarbon group may be saturated or unsaturated. In the case of an unsaturated aliphatic hydrocarbon group, the number of unsaturated bonds comprising the aliphatic hydrocarbon group is preferably 1 to 6, more preferably 1 to 3, and still more preferably 1 or 2. Examples of the unsaturated bond comprise a carbon-carbon double bond and a carbon-carbon triple bond, and the unsaturated bond is preferably a carbon-carbon double bond. The number of carbon atoms in the aliphatic hydrocarbon group is preferably 13 to 19, and more preferably 13 to 17. The aliphatic hydrocarbon group is not particularly limited, and examples thereof comprise an alkyl group, an alkenyl group, and an alkynyl group. Among these, the aliphatic hydrocarbon group is preferably an alkyl group or an alkenyl group. The aliphatic hydrocarbon group having 12 to 22 carbon atoms is not particularly limited, and examples thereof comprise a dodecyl group, a tridecyl group, a tetradecyl group, a pentadecyl group, a hexadecyl group, a heptadecyl group, an octadecyl group, a nonadecyl group, an icosyl group, a henicosyl group, a docosyl group, a dodecenyl group, a tridecenyl group, a tetradecenyl group, a pentadecenyl group, a hexadecenyl group, a heptadecenyl group, an octadecenyl group, a nonadecenyl group, an icosenyl group, a henicosenyl group, a docosenyl group, a dodecadienyl group, a tridecadienyl group, a tetradecadienyl group, a pentadecadienyl group, a hexadecadienyl group, a heptadecadienyl group, an octadecadienyl group, a nonadecadienyl group, an icosadienyl group, a henicosadienyl group, a docosadienyl group, an octadecatrienyl group, an icosatrienyl group, an icosatetraenyl group, an icosapentaenyl group, a docosahexaenyl group, an isostearyl group, a 1-hexylheptyl group, a 1-hexylnonyl group, a 1-octylnonyl group, a 1-octylundecyl group, and a 1-decylundecyl group. Among these, the aliphatic hydrocarbon group is preferably a tridecyl group, a pentadecyl group, a heptadecyl group, a nonadecyl group, a heptadecenyl group, a heptadecadienyl group, or a 1-hexylnonyl group, and more preferably a tridecyl group, a heptadecyl group, a heptadecenyl group, or a heptadecadienyl group.

In the present embodiment, the aliphatic hydrocarbon groups having 12 to 22 carbon atoms represented by R^{3a} and R^{3b} are preferably derived from a fatty acid. In this case, the carbonyl carbon derived from a fatty acid comprises -CO-O- in Formula (1). The aliphatic hydrocarbon group is not particularly limited, and is, for example, a heptadecadienyl group when linoleic acid is used as the fatty acid and a heptadecenyl group when oleic acid is used as the fatty acid.

R^{3a} may be the same as or different from R^{3b}, and although there are no particular limitations, it is preferable that R^{3a} is the same group as R^{3b}.

In the present embodiment, it is preferable that R^{1a} is the same as R^{1b}, X^{a} is the same as X^{b}, R^{2a} is the same as R^{2b}, Y^{a} is the same as Y^{b}, Z^{a} is the same as Z^{b}, and R^{3a} is the same as R^{3b}.

The cationic lipid represented by Formula (1) above is preferably any one of the following cationic lipids (1-1) to (1-3).

### [Cationic Lipid (1-1)]

A cationic lipid in which R^{1a} and R^{1b} are each independently an alkylene group having 1 to 6 carbon atoms (for example, a methylene group, an ethylene group);
X^{a} and X^{b} are each independently an acyclic alkyl tertiary amino group having one tertiary amino group wherein the alkyl has 1 to 6 carbon atoms (for example, - N(CH₃)-) or a cyclic alkylene tertiary amino group having one or two tertiary amino groups wherein the alkylene has 2 to 5 carbon atoms (for example, a piperidylene group);
R^{2a} and R^{2b} are each independently an alkylene group having 8 or less carbon atoms (for example, a methylene group, an ethylene group, a propylene group);
Y^{a} and Y^{b} are each independently an ester bond or an amide bond;
Z^{a} and Z^{b} are each independently a divalent group derived from an aromatic compound which may have a heteroatom, the aromatic compound having 3 to 16 carbon atoms and at least one aromatic ring (for example, -C₆H₄-CH₂-, -CH₂-C₆H₄-CH₂-); and
R^{3a} and R^{3b} are each independently a residue derived from a reaction product of a fat-soluble vitamin having a hydroxyl group (for example, tocopherol) and succinic anhydride or glutaric anhydride, or an aliphatic hydrocarbon group having 12 to 22 carbon atoms (for example, a heptadecenyl group, a heptadecadienyl group, a 1-hexylnonyl group).

### [Cationic Lipid (1-2)]

A cationic lipid in which R^{1a} and R^{1b} are each independently an alkylene group having 1 to 4 carbon atoms (for example, a methylene group, an ethylene group);
X^{a} and X^{b} are each independently an acyclic alkyl tertiary amino group having and one tertiary amino group wherein the alkyl has 1 to 3 carbon atoms (for example, -N(CH₃)-) or a cyclic alkylene tertiary amino group having 2 to 5 carbon atoms and one tertiary amino group (for example, a piperidylene group);
R^{2a} and R^{2b} are each independently an alkylene group having 6 or less carbon atoms (for example, a methylene group, an ethylene group, a propylene group);
Y^{a} and Y^{b} are each independently an ester bond or an amide bond;
Z^{a} and Z^{b} are each independently a divalent group derived from an aromatic compound which may have a heteroatom, the aromatic compound having 6 to 12 carbon atoms and one aromatic ring (for example, -C₆H₄-CH₂-, -CH₂-C₆H₄-CH₂-); and
R^{3a} and R^{3b} are each independently a residue derived from a reaction product of a fat-soluble vitamin having a hydroxyl group (for example, tocopherol) and succinic anhydride, or an aliphatic hydrocarbon group having 13 to 19 carbon atoms (for example, a heptadecenyl group, a heptadecadienyl group, a 1-hexylnonyl group).

### [Cationic Lipid (1-3)]

A cationic lipid in which R^{1a} and R^{1b} are each independently an alkylene group having 1 to 2 carbon atoms (for example, a methylene group, an ethylene group);
X^{a} and X^{b} are each independently X¹: 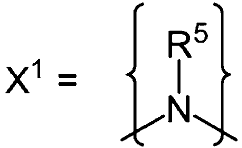
(in the formula, R⁵ is an alkyl group having 1 to 3 carbon atoms (for example, a methyl group)), or X²: 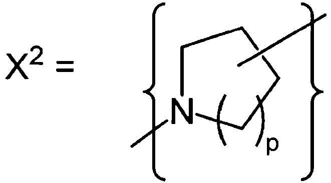
(in the formula, p is 1 or 2);
R^{2a} and R^{2b} are each independently an alkylene group having 4 or less carbon atoms (for example, a methylene group, an ethylene group, a propylene group);
Y^{a} and Y^{b} are each independently an ester bond or an amide bond;
Z^{a} and Z^{b} are each independently Z¹: 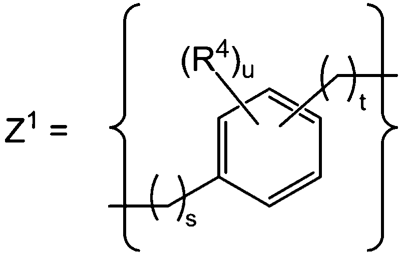
(in the formula, s is an integer of 0 or 1, t is an integer of 0 to 2, u is an integer of 0 to 2 (preferably 0), and R^{4'}s, of which the number is u, each independently represent a substituent.); and
R^{3a} and R^{3b} are each independently a residue derived from a reaction product of a fat-soluble vitamin having a hydroxyl group (for example, tocopherol) and succinic anhydride, or an aliphatic hydrocarbon group having 13 to 17 carbon atoms (for example, a heptadecenyl group, a heptadecadienyl group, a 1-hexylnonyl group).

In the present embodiment, the cationic lipid represented by Formula (1) above is not particularly limited, and examples thereof comprise O-Ph-P3C1, O-Ph-P4C1, O-Ph-P4C2, O-Bn-P4C2, E-Ph-P4C2, L-Ph-P4C2, HD-Ph-P4C2 and O-Ph-amide-P4C2, and O-Ph-C3M, among which O-Ph-P4C2 (hereinafter, also referred to as "ssPalmO") is preferable.

**[Table 1]**

| Name of cationic lipid | Structure |
|---|---|
| 0-Ph-P3C1 | 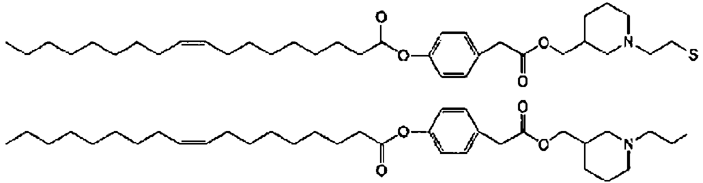 |
| 0-Ph-P4C1 | 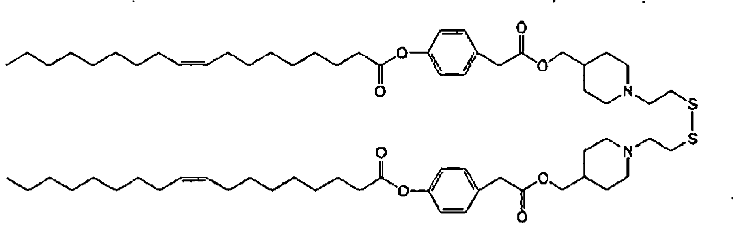 |
| 0-Ph-P4C2 | 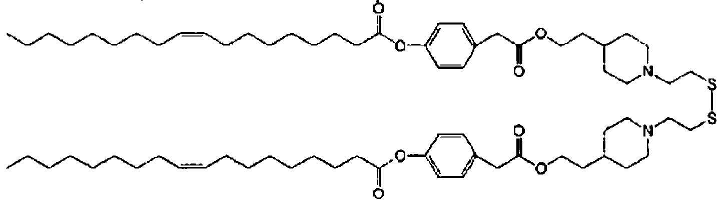 |
| 0-Bn-P4C2 | 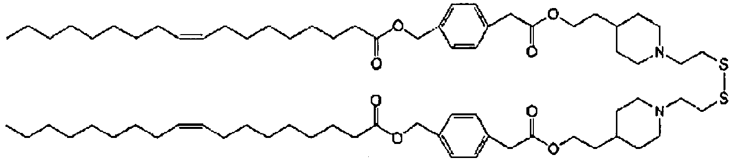 |
| E-Ph-P4C2 | 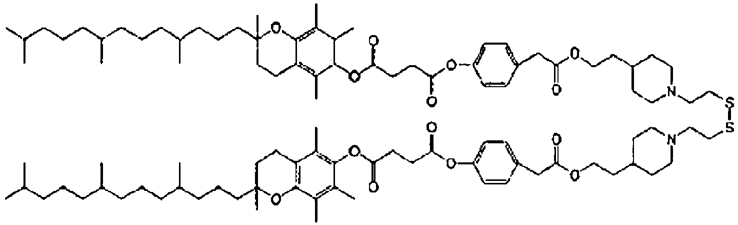 |

**[Table 2]**

| Name of cationic lipid | Structure |
|---|---|
| L-Ph-P4C2 | 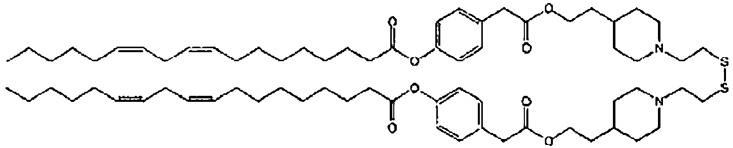 |
| HD-Ph-P4C2 | 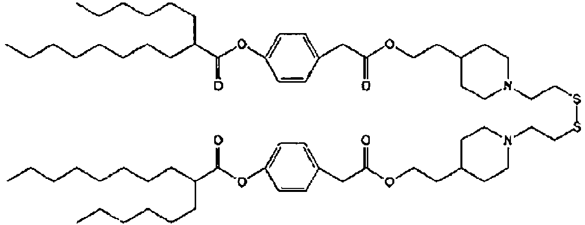 |
| 0-Ph-amide-P4C2 | 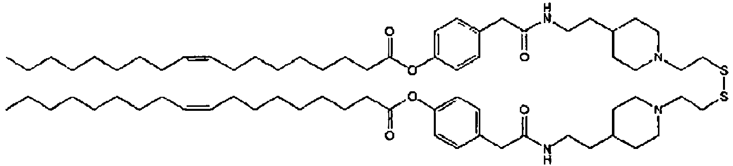 |
| 0-Ph-C3M | 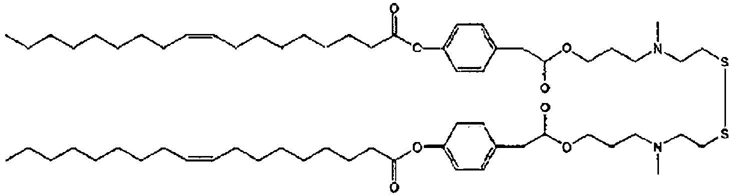 |

Although the ratio (mol%) of the cationic lipid represented by Formula (1) above to the total lipids present in the composition of the present embodiment is not particularly limited, the ratio is, for example, preferably 5% to 100%, more preferably 10% to 90%, still more preferably 20% to 70%, and even more preferably 35% to 65%. Among these, the ratio is preferably 40% to 60%, and more preferably 45% to 55%.

One of the cationic lipids represented by Formula (1) above may be used singly, or two or more thereof may be used in combination.

In the present embodiment, the cationic lipid represented by Formula (1) above can be produced by a known method. The method is not particularly limited, and the cationic lipid can be produced, for example, by the method described in WO 2019/188867.

The composition of the present embodiment may further comprise a nucleic acid. The nucleic acid is not particularly limited, and examples thereof comprise deoxyribonucleic acid (DNA) and ribonucleic acid (RNA), among which RNA is preferable. The RNA is not particularly limited, and examples thereof comprise mRNA, circular RNA, self amplifying RNA, miRNA, piRNA, tsRNA, siRNA, tRNA, rRNA, snRNA, snoRNA, and IncRNA. Among these, the RNA is preferably mRNA, circular RNA, self amplifying RNA, miRNA, piRNA, tsRNA, or siRNA, and more preferably mRNA, circular RNA, self amplifying RNA, or siRNA.

Although the nucleic acid is not particularly limited, a nucleic acid having preventive or therapeutic activity against a prescribed disease (preventive or therapeutic nucleic acid) is preferable from the viewpoint of use in a vaccine, and examples thereof comprise a nucleic acid derived from a pathogenic virus, a nucleic acid derived from pathogenic bacteria, a nucleic acid derived from a pathogenic fungus, and a nucleic acid derived from a pathogenic organism.

The pathogenic virus is not particularly limited, and examples thereof comprise influenza virus, avian influenza virus, parainfluenza virus, adenovirus, SARS coronavirus, MERS coronavirus, SARS coronavirus 2, AIDS virus, RS virus, cytomegalovirus, hepatitis virus, Japanese encephalitis virus, measles virus, rubella virus, varicella/herpes zoster virus, a virus belonging to the family Picornaviridae, a virus belonging to the family Papillomaviridae, herpes virus, mumps virus, rotavirus, cholera virus, rabies virus, and a virus that causes viral hemorrhagic fevers (for example, Ebola hemorrhagic fever, Marburg disease, Lassa fever, and Crimean-Congo hemorrhagic fever).

The pathogenic bacteria is not particularly limited, and examples thereof comprise diphtheria, tetanus, Mycobacterium tuberculosis, Streptococcus pneumoniae, Neisseria meningitidis, Staphylococcus, Pseudomonas aeruginosa, Bordetella pertussis, Bacillus anthracis, ricketsia, and salmonella.

The pathogenic fungus is not particularly limited, and examples thereof comprise Cryptococcus and Aspergillus.

The pathogenic organism is not particularly limited, and examples thereof comprise Plasmodium.

The nucleic acids also comprise nucleic acids derived from viruses, bacteria, fungi, and pathogenic organisms newly detected in the future.

One of the nucleic acids may be used singly, or two or more thereof may be used in combination. It is preferable that the nucleic acid is purified by a method commonly used by those skilled in the art.

In the present embodiment, nucleobases are not particularly limited, and may be, for example, the purine bases adenine (A) and guanine (G), and the pyrimidine bases thymine (T), cytosine (C), and uracil (U).

In the present embodiment, the nucleobases may be partially or fully modified as known in the technical field. The nucleobases may be, for example, modified nucleobases substituted with 1 to 3 arbitrary substituents (for example, a halogen atom, an alkyl group, an alkyl group substituted with an aromatic group, an alkoxy group, an acyl group, an alkoxyalkyl group, a hydroxy group, an amino group, a monoalkylamino group, a dialkylamino group, a carboxy group, an aryl group, a heteroaryl group, a cyano group, or a nitro group) at any positions (for example, an 8-bromoadenyl group, an 8-bromoguanyl group, a 5-bromocytosyl group, a 5-iodocytosyl group, a 5-bromouracil group, a 5-iodouracil group, a 5-fluorouracil group, a 5-methylcytosyl group, an 8-oxoguanyl group, or a hypoxanthinyl group).

In the present embodiment, the nucleobases may be partially or fully modified as known in the technical field. The modified nucleobases in the nucleic acids (for example, RNA nucleic acids such as mRNA nucleic acid) comprise 1-methyl-pseudouridine (m1Ψ), 1-ethyl-pseudouridine (e1Ψ), 5-methoxy-uridine (mo5U), 5-methyl-cytidine (m5C), and/or pseudouridine (Ψ). In some embodiments, the modified nucleobases in the nucleic acids (for example, RNA nucleic acids such as mRNA nucleic acid) comprise 5-methoxymethyluridine, 5-methylthiouridine, 1-methoxymethylpseudouridine, 5-methylcytidine and/or 5-methoxycytidine.

In the composition of the present embodiment, the ratio of the content of the cationic lipid represented by Formula (1) and the content of the nucleic acid may be 0.05 to 150, 0.05 to 145, 0.1 to 140, 0.2 to 135, 0.25 to 130, 0.5 to 125, 1 to 120, 5 to 115, 10 to 110, or 15 to 105, expressed as content of cationic lipid (nmol)/content of nucleic acid (µg).

When the total lipid content is within the above range, it is possible to maintain high vaccine effect while suppressing adverse reactions. The prophlogistic properties, which is the main cause of the adverse reactions, can be used as an indicator of the suppression of the adverse reactions, and a reduction in the amount of inflammatory cytokines and chemokines produced may also be used as an indicator.

The composition of the present embodiment may further comprise an additional component other than the cationic lipid represented by Formula (1) above and the nucleic acid.

Examples of the additional component comprise a lipid other than the cationic lipid represented by Formula (1) above, a surfactant, polyethylene glycol, and a protein.

The lipid other than the cationic lipid represented by Formula (1) above is not particularly limited, and examples thereof comprise an ionizable lipid, a neutral lipid, a PEG-modified lipid, and a sterol.

The ionizable lipid is not particularly limited, and can be a lipid having a positive charge at a selected pH, such as a physiological pH, or the like. Examples of the ionizable lipid comprise 1,2-dilinoleyloxy-3-dimethylaminopropane (DLin-DMA), dilinoleylmethyl-4-dimethylaminobutyrate (DLin-MC3-DMA) (for example, see U.S. Patent Specification No. 8,158,601), 2-dilinoleyl-4-dimethylaminoethyl-[1,3]-dioxolane (DLin-KC2-DMA), Merck-32 (for example, see WO 2012/018754), Acuitas-5 (for example, see WO 2015/199952), KL-10 (for example, see U.S. Patent Application Publication No. 2012/0295832), C12-200 (for example, see Love, KT et al., PNAS, 107: 1864 (2009)), and an amino alcohol lipid.

The neutral lipid is not particularly limited, and examples thereof comprise a lipid that does not have a net positive charge at a selected pH such as a physiological pH. Neutral lipids are also referred to as helper lipids or neutral phospholipids. The neutral lipid is not particularly limited, and examples thereof comprise distearoylphosphatidylcholine (DSPC), dioleoylphosphatidylcholine (DOPC), dipalmitoylphosphatidylcholine (DPPC), dioleoyl-phosphatidylethanolamine (DOPE), palmitoyloleoylphosphatidylcholine (POPC), palmitoyloleoyl-phosphatidylethanolamine (POPE), and dioleoylphosphatidylethanolamine 4-(N-maleimidomethyl)-cyclohexane-1-carboxylate (DOPE-mal). Among these, the neutral lipid is preferably DOPC, DPPC, DSPC, POPC, or DOPE, and more preferably DOPC or DSPC.

The content of the neutral lipid in the composition of the present embodiment may be 1 mol% to 20 mol%, and is preferably 5 mol% to 15 mol%.

The PEG-modified lipid is not particularly limited, and can be a lipid comprising a polymer moiety such as polyethylene glycol. Examples of the PEG-modified lipid comprise dimyristoylphosphatidylethanolamine-poly(ethylene glycol) 2000 (DMPE-PEG2000), DPPE-PEG2000, DMG-PEG2000, DPG-PEG2000, PEG2000-c-DOMG, and PEG2000-c-DOPG. Among these, the PEG-modified lipid is preferably DMG-PEG2000. The molecular weight of polyethylene glycol is not particularly limited, and can be, for example, in a range of about 500 to about 10,000 Da. The molecular weight is preferably in a range of about 1,000 to about 5,000 Da, and specific examples of polyethylene glycol comprise DMG-PEG1000, DMG-PEG2000, and DMG-PEG5000.

The content of the PEG-modified lipid in the composition of the present embodiment may be 0.01 mol% to 10 mol%, and is preferably 0.1 mol% to 5 mol%.

The sterol is not particularly limited, and examples thereof comprise cholesterol, cholestanol, stigmasterol, β-sitosterol, lanosterol, and ergosterol. Among these, the sterol is preferably cholesterol, cholestanol, stigmasterol, or β-sitosterol.

The content of the sterol in the composition of the present embodiment may be 10 mol% to 60 mol%, and is preferably 20 mol% to 50 mol%.

One of the lipids other than the cationic lipid represented by Formula (1) above may be used singly, or two or more thereof may be used in combination.

In the composition of the present embodiment, the ratio of the total content of the cationic lipid represented by Formula (1) and the lipid other than the cationic lipid represented by Formula (1) (hereinafter, also referred to as "total lipid content") and the content of the nucleic acid may be 0.05 to 250, 0.05 to 245, 0.1 to 240, 0.5 to 235, 1 to 230, 5 to 225, 10 to 220, 15 to 215, 25 to 210, or 30 to 205, expressed as total lipid content (nmol)/content of nucleic acid (µg).

When the total lipid content is within the above range, it is possible to maintain high vaccine effect while suppressing adverse reactions. The prophlogistic properties, which is the main cause of the adverse reactions, can be used as an indicator of the suppression of the adverse reactions, and a reduction in the amount of inflammatory cytokines and chemokines produced may also be used as an indicator.

The composition of the present embodiment may be a lipid membrane structure comprising the cationic lipid represented by Formula (1) above as a component of the membrane, from the viewpoint of use in a vaccine. Here, the "lipid membrane structure" refers to a particle having a membrane structure in which the hydrophilic groups of amphipathic lipids are aligned toward the aqueous phase at the interface. In addition, it is preferable that the lipid membrane structure encapsulates the nucleic acid.

The form of the lipid membrane structure is not particularly limited, and examples thereof comprise liposomes (for example, unilamellar liposomes and multilamellar liposomes), an O/W emulsion, a W/O emulsion, spherical micelles, wormlike micelles, LNPs, and unspecified lamellar structures. Among these, the lipid membrane structure is preferably in the form of LNPs.

The composition of the present embodiment is not particularly limited, and can be prepared, for example, by dispersing the cationic lipid represented by Formula (1) above and the additional components (for example, the lipid other than the cationic lipid represented by Formula (1) above) in a suitable solvent or dispersion medium (for example, an aqueous solvent or an alcoholic solvent) and performing an operation of inducing assembly, as necessary.

As the operation of inducing assembly, a known method can be used. Although the method is not particularly limited, examples thereof comprise an ethanol dilution method using a microchannel or a vortex, a simple hydration method, an ultrasonic treatment, heating, vortexing, an ether infusion method, a French press method, a cholic acid method, a Ca²⁺ fusion method, a freeze-thawing method, and a reverse-phase evaporation method.

In a case where the composition of the present embodiment is the lipid membrane structure and further comprises the nucleic acid, the lipid membrane structure encapsulating the nucleic acid can be prepared by adding the target nucleic acid during the formation of the composition of the present embodiment. Although the method is not particularly limited, in a case where liposomes are formed by, for example, the ethanol dilution method, an aqueous solution of the nucleic acid and an ethanol solution of the components of the composition of the present embodiment (for example, the cationic lipid represented by Formula (1) above and other additional components) may be vigorously mixed in a vortex, a microchannel, or the like, and then the mixture may be diluted with a suitable buffer. In a case where liposomes are formed by the simple hydration method, a thin lipid film is obtained by dissolving the components of the composition of the present embodiment in a suitable organic solvent, pouring the solution into a glass container, and distilling the solvent by drying under reduced pressure. After hydrating the obtained thin lipid film by adding an aqueous solution of the nucleic acid, the thin lipid film may be subjected to an ultrasonic treatment using a sonicator. Therefore, the composition of the present embodiment may be a lipid membrane structure encapsulating the nucleic acid.

In the present specification, the term "vaccine" is an art-recognized term and refers to a preparation for preventing (preventive vaccine) or treating (therapeutic vaccine) a disease by increasing immunity against the disease. Therefore, when the composition of the present embodiment further comprises the nucleic acid, the composition may be used to prevent or treat an infectious disease.

In the present specification, the term "to prevent or to treat" refers to preventative and/or therapeutic measure. The term "preventive or therapeutic measure" is an art-recognized term and may comprise administration of the composition of the present embodiment to a subject.

The therapeutic measure may refer to the administration after the finding of an undesirable condition (for example, a disease or other undesirable condition of the subject) and may be intended to reduce, suppress, recover from, alleviate, or stabilize an existing undesirable condition or an adverse reaction thereof. The therapeutic measure also comprises the attenuation of a direct or indirect pathological effect of a disease and prevention of metastasis.

The therapeutic measure may also refer to a treatment for delaying the onset of a disease or slowing down the progression of a disease.

The preventive measure may refer to the administration before the clinical finding of an undesirable condition and may be intended for the protection against an undesirable condition (for example, an infectious disease) and the suppression and/or prevention of a recurrence of the undesirable condition. The prevention of a disease may refer to inducing an immune defense in the subject. The induction of the immune defense refers to the induction of immunity or an immune response against a disease agent (for example, an infectious agent), which is presented by a vertebrate (for example, a human). The induction of the immune defense can prevent or ameliorate an infection or relieve at least one symptom thereof.

The infectious disease is not particularly limited, and examples thereof comprise an infectious disease caused by the pathogenic virus, the pathogenic bacteria, the pathogenic fungus, or the pathogenic organism described above.

The composition of the present embodiment is preferably, for example, for oral administration or parenteral administration (for example, intravenous administration, intramuscular administration, topical administration, transnasal administration, transdermal administration, subcutaneous administration, intradermal administration, and intraperitoneal administration), more preferably for parenteral administration, still more preferably for intradermal administration, subcutaneous administration, transnasal administration, or intramuscular administration, and even more preferably for subcutaneous administration or intramuscular administration, but the use of the composition is not limited thereto.

The subject to which the composition of the present embodiment is administered is a mammal. The mammal is not particularly limited, and examples thereof comprise a human, a non-human primate, a domestic animal, an experimental animal, and livestock, with a human being preferred as the mammal. In addition, it is preferable that the composition is administered to a healthy subject.

When the composition of the present embodiment is administered, the composition of the present embodiment may reach and contact the target cells, and the nucleic acid comprised in the present embodiment may be introduced into the cells in the living body.

The dose and administration interval for the composition of the present embodiment can be appropriately selected according to the subject to be administered, the route of administration, and the age, weight, and symptom of the subject.

Although the dose is not particularly limited, in the case of parenteral administration, the dose per administration for adults is, for example, 0.01 µg to 100 mg, and may be 0.1 µg to 10 mg or 0.1 µg to 1 mg. As for the administration interval for the composition of the present embodiment, a single dose may be administered once a day or in divided doses several times a day.

### [Examples]

Examples of the present invention are presented below in order to describe the present invention in further detail, but the present invention is not limited thereto.

### Experimental Animals, Reagents, and the like

- Mice: 6-week-old male C57BL6 mice
- mRNA: DNAs of hemagglutinin (HA) and neuraminidase (NA) derived from A/Viet Nam/1203/2004 (H5N1) were amplified by PCR to prepare plasmid DNA serving as a template for mRNA. The plasmid was linearized using a restriction enzyme and purified by phenol-chloroform extraction and ethanol precipitation. The linearized plasmid was transcribed using MEGAscript T7 transcription kit (Thermo Fisher Scientific Inc.), thereby obtaining mRNA. Note that uridine in the mRNA was replaced with N1-methylpseudouridine. After removing residual double-stranded RNA from the prepared mRNA, 5' Cap modification was added in accordance with the protocol of ScriptCap Cap 1 Capping System (C-SCCS1710, Madison, WI, USA), and 3' end poly(A) tail modification was added in accordance with the protocol of poly(A) Tailing Kit (AM1350, Thermo Fisher Scientific Inc.).
- LNPs: LNP_{ssPalmO-1}, LNP_{ssPalmO-2}, and LNPSM102

The ratio of the amounts of a nucleic acid and a lipid is defined as the Lipid/RNA ratio (L/R ratio (nmol/µg)), which is the amount of the lipid (nmol) relative to the weight of the nucleic acid (µg). LNPs were prepared with the following lipid composition ratios and L/R ratios.
LNP_{ssPalmO-1}: ssPalmO/DOPC/cholesterol/DMG-PEG2000 = 52.5/7.5/40/1.5, L/R ratio = 200
LNP_{ssPalmO-2}: ssPalmO/DOPC/cholesterol/DMG-PEG2000 = 52.5/7.5/40/1.5, L/R ratio = 33
LNP_{SM102}: SM-102/DSPC/cholesterol/DMG-PEG2000 = 50/10/38.5/1.5, L/R ratio = 33

Note that ssPalmO is "COATSOME (registered trademark) SS-OP" purchased from NOF CORPORATION. SM-102 is (formal name: 8-[(2-hydroxyethyl)[6-oxo-6-(undecyloxy)hexyl]amino]-octanoic acid, 1-octylnonyl ester) purchased from Cayman Chemical Company.

The LNPs were prepared as follows.
- LNP_{ssPalmO-1}: Ethanol solutions of the lipids were mixed so that the total lipid concentration of ssPalmO, DOPC, and cholesterol was 4.0 mM. The mRNA solution was diluted using a malic acid/NaOH buffer (pH 3.0, 30 mM NaCl) so that the mRNA concentration was 0.0067 mg/mL. The two solutions were mixed using NanoAssemblr Ignite (Precision Nanosystems Inc., Vancouver, Canada) so that the ratio of the nucleic acid solution and the lipid solution was 3:1. The total flow rate of the solutions at this time was 4.0 mL/min. The solution was mixed with an equal amount of MES buffer (pH 6.5) and subjected to ultrafiltration at room temperature at 1000 g. PBS was added to the particle solution, and the solution was subjected to ultrafiltration. A particle solution was obtained by adding PBS to the obtained particle solution again and performing ultrafiltration.
- LNP_{ssPalmO-2}: Ethanol solutions of the lipids were mixed so that the total lipid concentration of ssPalmO, DOPC, and cholesterol was 1.2 mM. The mRNA solution was diluted using a malic acid/NaOH buffer (pH 3.0, 30 mM NaCl) so that the mRNA concentration was 0.012 mg/mL. The two solutions were mixed using NanoAssemblr Ignite (Precision Nanosystems Inc., Vancouver, Canada) so that the ratio of the nucleic acid solution and the lipid solution was 3:1. The total flow rate of the solutions at this time was 4.0 mL/min. The solution was mixed with an equal amount of MES buffer (pH 6.5) and subjected to ultrafiltration at room temperature at 1000 g. PBS was added to the particle solution, and the solution was subjected to ultrafiltration. A particle solution was obtained by adding PBS to the obtained particle solution again and performing ultrafiltration.
- LNP_{SM-102}: Ethanol solutions of the lipids were mixed so that the total lipid concentration of SM-102, DSPC, cholesterol, and DMG-PEG2000 was 1.2 mM. The mRNA solution was diluted using an acetic acid/NaOH buffer (pH 5.0) so that the mRNA concentration was 0.012 mg/mL. The two solutions were mixed using NanoAssemblr Ignite (Precision Nanosystems Inc., Vancouver, Canada) so that the ratio of the nucleic acid solution and the lipid solution was 3:1. The total flow rate of the solutions at this time was 2.0 mL/min. The solution was mixed with an equal amount of MES buffer (pH 5.5) and subjected to ultrafiltration at room temperature at 1000 g. PBS was added to the particle solution, and the solution was subjected to ultrafiltration. A particle solution was obtained by adding PBS to the obtained particle solution again and performing ultrafiltration.

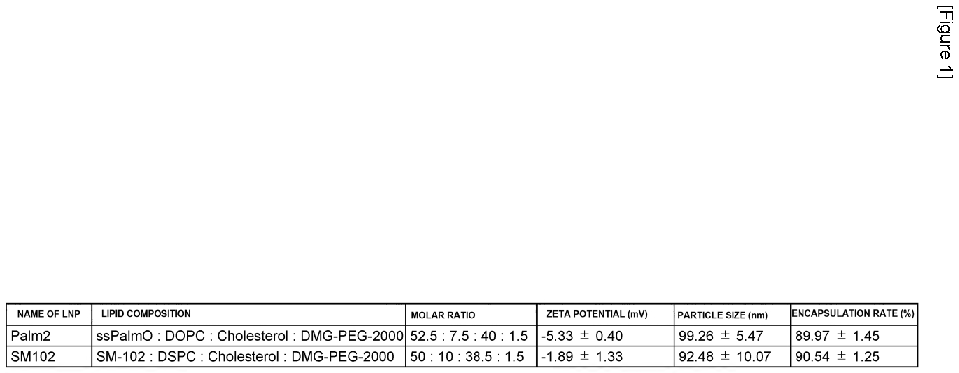

The particle sizes and zeta potentials of the mRNA-LNPs were measured with Zetasizer Nano ZS (Malvern Instruments Ltd., Malvern, UK), and the encapsulation rates of the mRNA-LNPs were measured with Quant-it (registered trademark) RiboGreen RNA Assay Kit (Thermo Fisher Scientific Inc.). The measurement results are shown in Figure. 1.

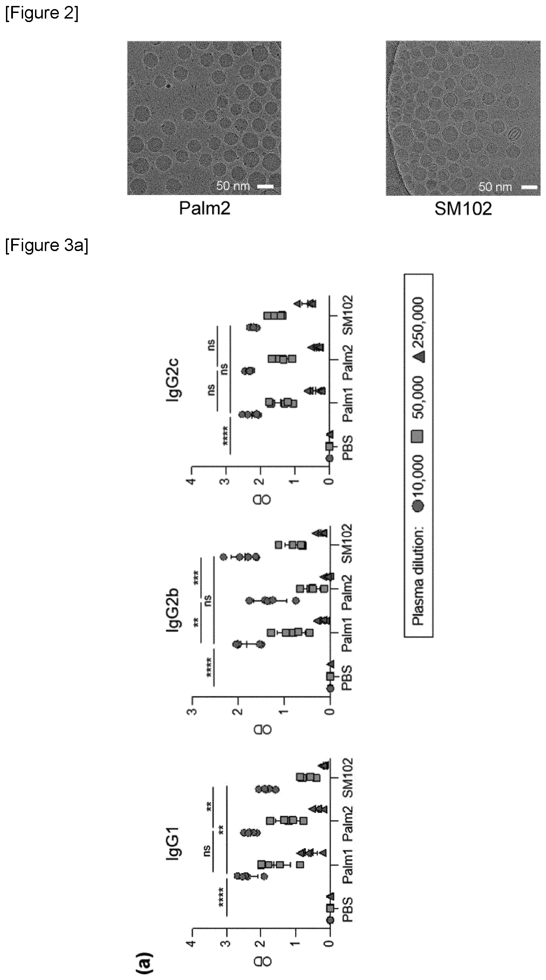

The results of observing the prepared mRNA-LNPs with a cryo-electron microscope (Talos Arctica, FEI) are shown in Figure 2.
- Antigens for ELISA: Recombinant proteins of HA and NA derived from A/VietNam/1203/2004 (H5N1) (denoted by rHA and rNA)

### (Test Example 1 Evaluation of Antibody Titers after Two Subcutaneous Immunizations)

The mice were subcutaneously immunized with HA- or NA-derived mRNA-LNPs (1 µg/mouse as the amount of mRNA) on days 0 and 21. Blood and draining lymph nodes were collected on day 35. rHA- and rNA-specific IgG1, IgG2b, and IgG2c in the plasma were evaluated by ELISA. Using a 2-fold serial dilution series, antibody titers were calculated for the rHA-specific IgG1, IgG2b, and IgG2c in the plasma. Germinal center B cells were detected by a flow cytometer (Attune NxT, Thermo Fisher Scientific Inc.).

A hemagglutination test (HI test) was performed by the following steps. After pretreating the serum with RDE (II) receptor-destroying enzyme (DENKA Co., Ltd., Tokyo, Japan), the serum was diluted 10-fold and then serially diluted 2-fold. After allowing the serum to react with 8 HA units of viral antigen for 30 minutes, the serum was mixed with chicken erythrocytes (Japan Bioserum Co., Ltd., Hiroshima, Japan) and incubated for 30 minutes. The maximum serum dilution factor that completely inhibited hemagglutination was taken as the HI antibody titer. The HI test evaluated the HI antibody titers against the Vietnam strain (A/Viet Nam/1203/2004), which was the same as the vaccine strain, and the Hokkaido strain (A/Ezo red fox/Hokkaido/1/2022), which was an H5N1 virus different from the vaccine strain. The Vietnam strain and the Hokkaido strain share 91% homology in HA and 94% homology in NA at the amino acid level.

In the figures described below, LNP_{ssPalmO-1}, LNP_{ssPalmO-2}, and LNP_{SM102} are referred to as Palm1, Palm2, and SM102, respectively.

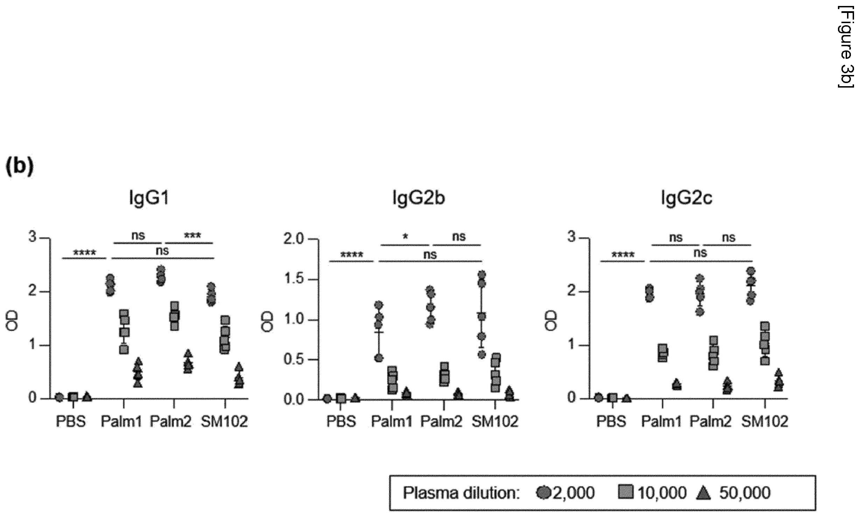

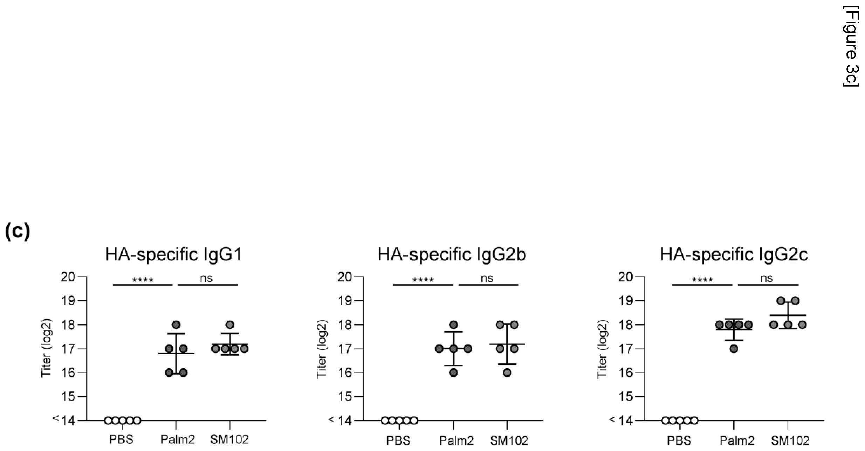

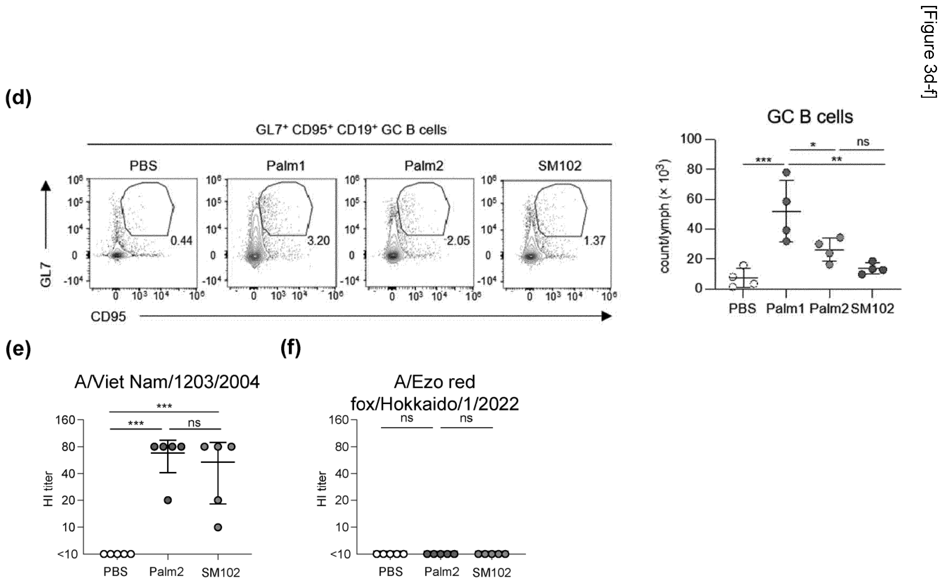

The results of evaluating the rHA- and rNA-specific IgGs in the plasma are shown in Figure 3. (a) shows the results of rHA-specific IgG induction in the group subcutaneously immunized twice with the HA-derived mRNA, and (b) shows the results of rNA-specific IgG induction in the group subcutaneously immunized twice with the NA-derived mRNA. For both HA and NA, higher antibody titers were observed in the mRNA-LNP group compared to the non-vaccine group. Although significant differences were observed between the LNPs, the differences were not remarkable. From the above results, it was determined that LNP_{ssPalmO-1} and LNP_{ssPalmO-2} induced antigen-specific antibody titers comparable to those induced by LNP_{SM102}.

The antibody titers of the rHA-specific IgGs in the plasma are shown in (c). Higher antibody titers were observed in the group subcutaneously immunized twice with the HA-derived mRNA compared to the non-vaccine group. No significant differences were observed between the LNPs.

The number of germinal center B cells in the draining lymph nodes are shown in (d). LNP_{ssPalmO-1} and LNP_{ssPalmO-2}, which were the groups subcutaneously immunized twice with the HA-derived mRNA, tended to induce the germinal center B cells more potently than LNP_{SM102}.

The HI antibody titers against the Vietnam strain and Hokkaido strain are shown in (e) and (f). The group subcutaneously immunized twice with the HA-derived mRNA showed higher HI antibody titers against the Vietnam strain compared to the non-vaccine group, and no significant differences were observed between the LNPs. Since the Hokkaido strain was different from the vaccine strain, the HI antibody titers against the Hokkaido strain were not observed in the group subcutaneously immunized twice with the HA-derived mRNA.

From the results of the present test example, it was shown that LNP_{ssPalmO-2} induced antigen-specific antibody titers comparable to those induced by LNP_{SM102}.

### (Test Example 2 Evaluation of Antibody Titers after Subcutaneous Prime Immunization)

The mice were subcutaneously immunized with HA- or NA-derived mRNA-LNPs (1 µg/mouse as the amount of mRNA) on day 0. On day 14, blood was collected, and rHA- and rNA-specific IgG1, IgG2b, and IgG2c in the plasma were evaluated by ELISA.

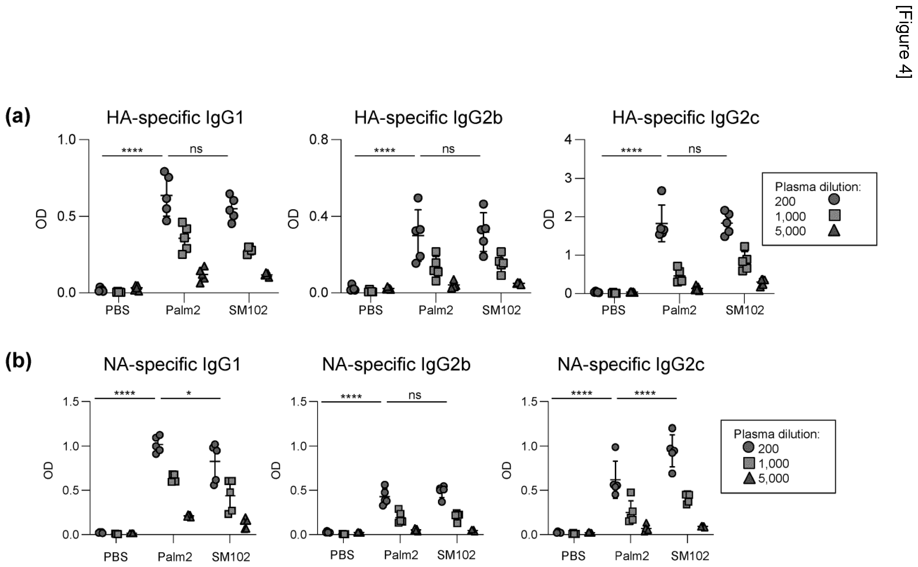

The results of evaluating the rHA- and rNA-specific IgGs in the plasma are shown in Figure 4. (a) shows the results of rHA-specific IgG induction in the group subcutaneously immunized once with the HA-derived mRNA, and (b) shows the results of rNA-specific IgG induction in the group subcutaneously immunized once with the NA-derived mRNA. The antibody titers of the rHA-specific IgGs were observed to be higher in the mRNA-LNP group compared to the non-vaccine group, and no significant differences were observed between the LNPs. The antibody titers of the rNA-specific IgGs were observed to be higher in the mRNA-LNP group compared to the non-vaccine group. Although significant differences were observed between the LNPs, the differences were not remarkable.

From the results of the present test example, it was shown that LNP_{ssPalmO-2} induced antigen-specific antibody titers comparable to those induced by LNP_{SM102}.

### (Test Example 3 Comparison with Recombinant Protein + alum)

The mice were subcutaneously immunized with HA-derived mRNA-LNPs (1 µg/mouse as the amount of mRNA) on days 0 and 21. Also, the mice were subcutaneously immunized with rHA (recombinant HA protein, 1 µg/mouse) + alum (aluminum hydroxide salt, 50 µg/mouse) as controls. Blood and spleens were collected on day 35. rHA-specific IgG1, IgG2b, and IgG2c in the plasma were evaluated by ELISA. The spleens were restimulated in vitro with rHA, and then IFN-y- or IL-13-producing CD4- and CD8-positive T cells were detected by intracellular staining.

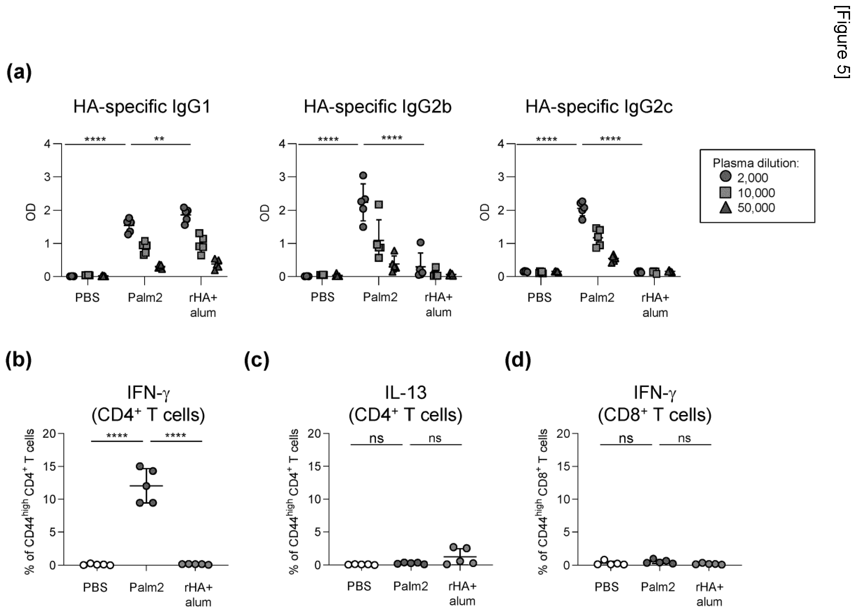

The results of evaluating the rHA-specific IgGs in the plasma are shown in Figure 5. (a) shows the results of rHA-specific IgG induction in the group subcutaneously immunized twice with the HA-derived mRNA or rHA + alum. Higher antibody titers were observed in the mRNA-LNP group compared to the non-vaccine group. The induction of IgG2b and IgG2c by the mRNA-LNPs was significantly higher compared to the induction in the rHA + alum vaccine group.

(b) to (d) show the results of T cell responses in the group subcutaneously immunized twice with the HA- derived mRNA or rHA + alum. The IFN-γ-producing CD4-positive T cells were significantly induced in the mRNA-LNP vaccine group compared to the non-vaccine group, and the IFN-γ-producing CD4-positive T cells were not induced in the rHA + alum vaccine group. On the other hand, the IL-13-producing CD4-positive T cells and the IFN-γ-producing CD8-positive T cells were not induced in any of the vaccine groups, when compared with the non-vaccine group.

From the results of the present test example, it was determined that the antibody induction and the IFN-γ-producing CD4-positive T cell induction were superior with LNP_{ssPalmO-2} compared to rHA + alum.

### (Test Example 4 Evaluation of Antibody Titers after Two Intramuscular Immunizations)

The mice were intramuscularly immunized with HA-derived mRNA-LNPs (1 µg/mouse as the amount of mRNA) on days 0 and 21. On day 35, blood was collected, and rHA-specific IgG1, IgG2b, and IgG2c in the plasma were evaluated by ELISA.

The hemagglutination test (HI test) evaluated the HI antibody titers against the Vietnam strain (A/Viet Nam/1203/2004), which was the same as the vaccine strain, and the Hokkaido strain (A/Ezo red fox/Hokkaido/1/2022), which was an H5N1 virus different from the vaccine strain.

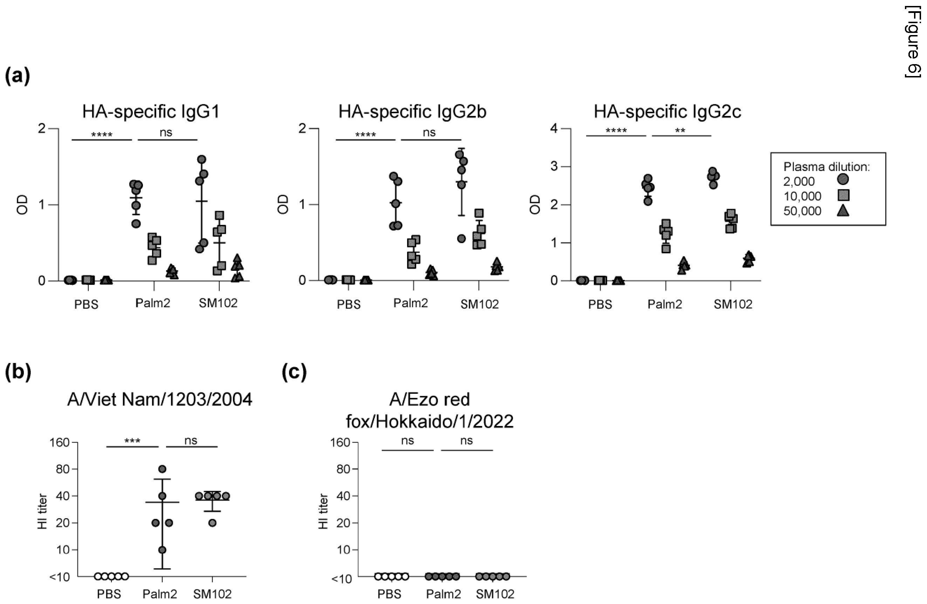

The results of evaluating the rHA-specific IgGs in the plasma are shown in Figure 6(a). Higher antibody titers were observed in the group intramuscularly immunized twice with the HA-derived mRNA compared to the non-vaccine group. Although significant differences were observed between the LNPs, the differences were not remarkable.

The HI antibody titers against the Vietnam strain and Hokkaido strain are shown in Figures 6(b) and (c). The group intramuscularly immunized twice with the HA-derived mRNA showed higher HI antibody titers against the Vietnam strain compared to the non-vaccine group, and no significant differences were observed between the LNPs. Since the Hokkaido strain was different from the vaccine strain, the HI antibody titers against the Hokkaido strain were not observed in the group intramuscularly immunized twice with the HA-derived mRNA.

From the results of the present test example, it was determined that LNP_{ssPalmO-2} induced antigen-specific antibody titers comparable to those induced by LNP_{SM102} even when administered intramuscularly.

### (Test Example 5 T Cell Responses)

The mice were subcutaneously or intramuscularly immunized with HA- or NA-derived mRNA-LNPs (1 µg/mouse as the amount of mRNA) on days 0 and 21. Spleens were collected on day 35. The spleens were restimulated in vitro with rHA or rNA, and then IFN-γ- or IL-13-producing CD4- and CD8-positive T cells were detected by intracellular staining.

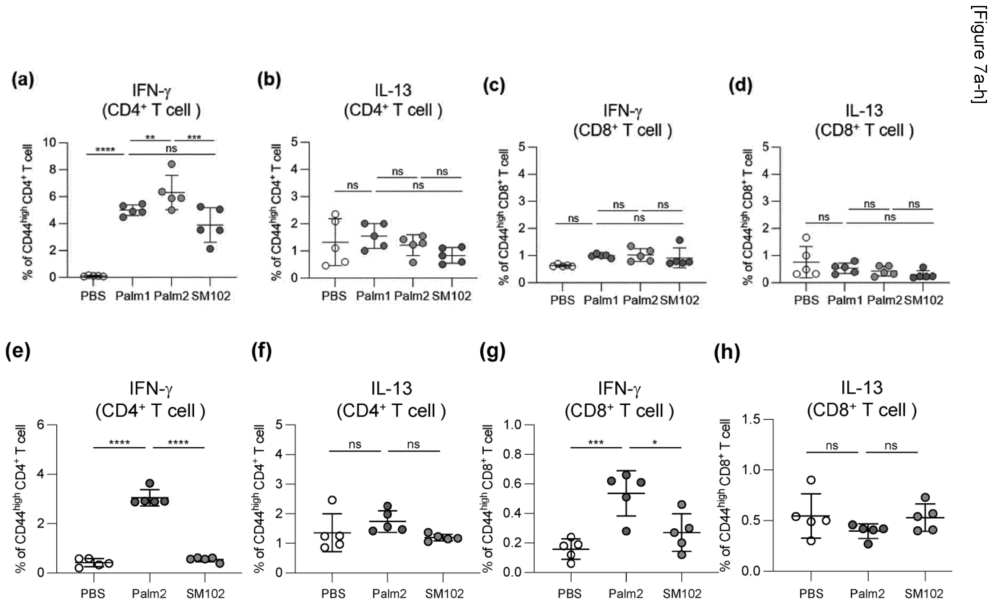

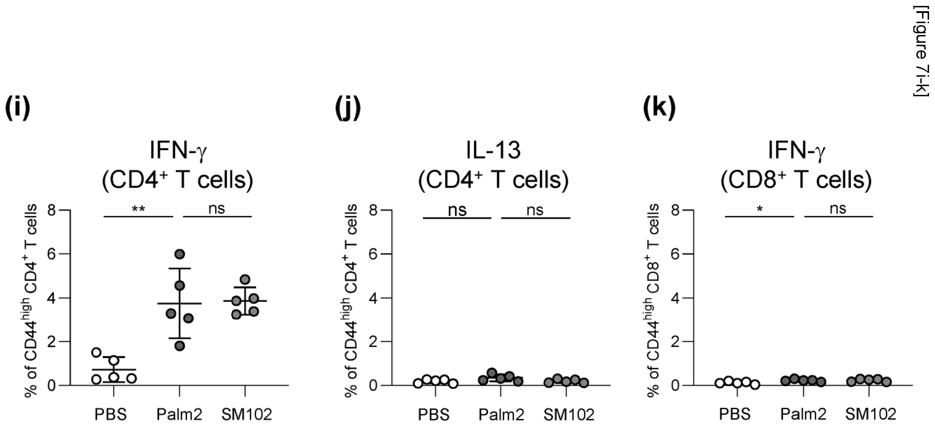

The results of T cell responses are shown in Figure 7. (a) to (d) show the results of T cell responses in the group subcutaneously immunized twice with the HA-derived mRNA. The IFN-γ-producing CD4-positive T cells were significantly induced in the mRNA-LNP vaccine groups compared to the non-vaccine group, and LNP_{ssPalmO-2} significantly induced the IFN-γ-producing CD4-positive T cells compared to LNP_{ssPalmO-1} and LNP_{SM102}. On the other hand, the IL-13-producing CD4-positive T cells, the IFN-γ-producing CD8-positive T cells, and the IL-13-producing CD8-positive T cells were not induced in any of the vaccine groups compared to the non-vaccine group.

(e) to (h) show the results of T cell responses in the group subcutaneously immunized twice with the NA-derived mRNA. The IFN-γ-producing CD4-positive T cells and the IFN-γ-producing CD8-positive T cells were significantly induced in the LNP_{ssPalmO-2} group compared to the non-vaccine group, and these cells were not induced in the LNP_{SM102}. On the other hand, the IL-13-producing CD4-positive T cells and the IL-13-producing CD8-positive T cells were not induced in any of the vaccine groups compared to the non-vaccine group.

(i) to (k) show the results of T cell responses in the group intramuscularly immunized twice with the HA-derived mRNA. The IFN-γ-producing CD4-positive T cells were significantly induced in the mRNA-LNP vaccine groups compared to the non-vaccine group, and no significant differences were observed between the LNPs. On the other hand, the IL-13-producing CD4-positive T cells were not induced in any of the vaccine groups compared to the non-vaccine group. The IFN-γ-producing CD8-positive T cells were significantly induced in the LNP_{ssPalmO-2} group compared to the non-vaccine group, and these cells were not induced in the LNP_{SM102} group.

From the results of the present test example, it was determined that the level of the IFN-γ-producing CD4-positive T cell induction by LNP_{ssPalmO-2} was comparable to or greater than the level of the induction in the LNP_{SM102} group.

### (Test Example 6 Evaluation of Ability to Protect against Infection Using Body Weight and Survival Rate as Indicators)

The mice were subcutaneously immunized with HA- or NA-derived mRNA-LNPs (1 µg/mouse as the amount of mRNA) on days 0 and 21. On day 35, the mice were transnasally infected with the Vietnam strain (A/Viet Nam/1203/2004), which was the same the vaccine strain, and then the ability to protect against the infection was evaluated using the body weight and the survival rate as indicators. In addition, in order to evaluate the cross-protection ability, the mice were transnasally infected with the Hokkaido strain (A/Ezo red fox/Hokkaido/1/2022), which was an H5N1 virus different from the vaccine strain, or the California strain (A/California/07/2009), which was an H1N1 virus, and then the ability to protect against the infection was evaluated using the body weight and the survival rate as the indicators.

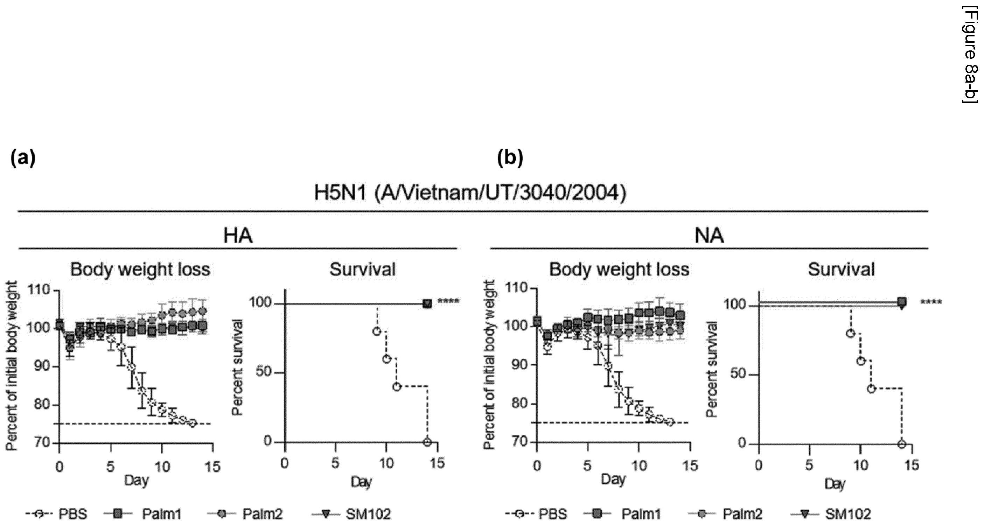

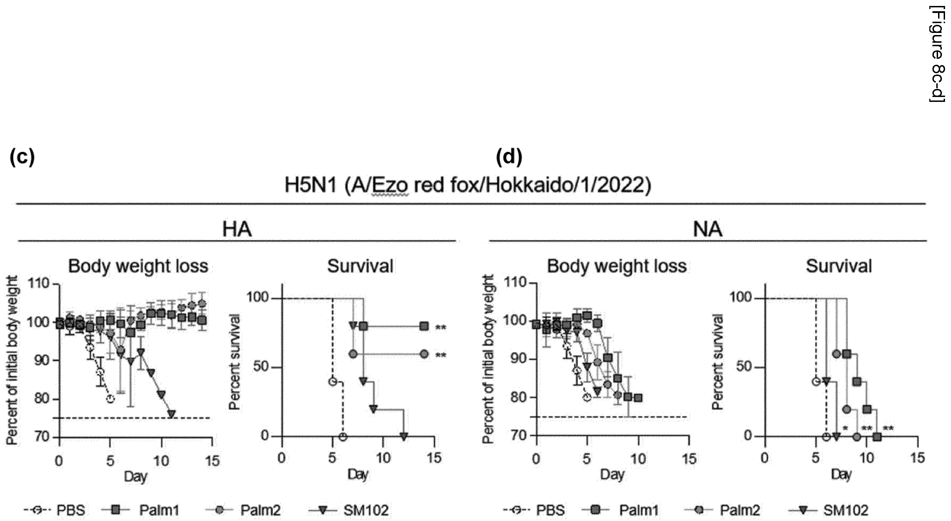

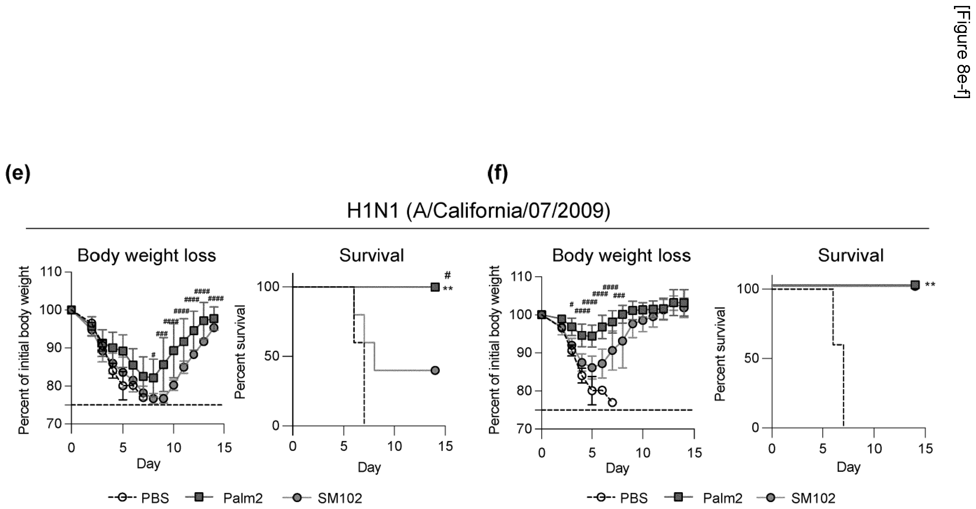

The results of the body weight and the survival rates are shown in Figure 8. (a), (c), and (e) show the group subcutaneously immunized twice with the HA-derived mRNA-LNPs, and (b), (d), and (f) show the group subcutaneously immunized twice with the NA-derived mRNA-LNPs.

(a) and (b) are the results of observing the body weights and the survival rates of the mice infected with the Vietnam strain. In the non-vaccine group, significant body weight loss was observed, and all mice died within 14 days. On the other hand, the body weight loss was not observed with any of the LNPs, regardless of whether the LNPs were derived from HA or NA, and all mice survived. From the above results, it was determined that LNP_{ssPalmO-1} and LNP_{ssPalmO-2} can induce an effect of protecting against an infection that is comparable to that induced by LNP_{SM102}.

(c) and (d) are the results of observing the body weights and the survival rates of the mice infected with the Hokkaido strain. In the mice that used HA as the antigen, the survival rate tended to be longer with LNP_{SM102} compared to the survival rate in the non-vaccine group, but all mice died within 14 days of the infection. Meanwhile, in the cases of LNP_{ssPalmO-1} and LNP_{ssPalmO-2}, the survival rates were 80% and 60%, respectively. That is, it was shown that the cross-protection ability of LNP_{ssPalmO-1} and LNP_{ssPalmO-2} may be superior to that of LNP_{SM102}. In addition, all of the mice that used NA as the antigen died within 14 days of the infection regardless of the type of LNPs, but slight increases in the survival rate were observed, with the effect increasing in the order of LNP_{ssPalmO-1}, LNP_{ssPalmO-2}, and LNP_{SM102}. From the above results, it was shown that the cross-protection ability against the Hokkaido strain may be superior with HA than NA, and the cross-protection abilities of LNP_{ssPalmO-1} and LNP_{ssPalmO-2} may be superior to that of LNP_{SM102}.

(e) and (f) are the results of observing the body weights and the survival rates of the mice infected with the California strain. In the mice that used HA as the antigen, the survival rate tended to be longer with LNP_{SM102} compared to the survival rate in the non-vaccine group, but the survival rate was still 40%. On the other hand, in the case of LNP_{ssPalmO-2}, the survival rate was 100%. Furthermore, all of the mice that used NA as the antigen survived regardless of the type of LNPs, but the body weight loss was smaller with LNP_{ssPalmO-2} than with LNP_{SM102}. From the above results, it was shown that the cross-protection ability against the California strain may be superior with NA than HA, and the cross-protection abilities of LNP_{ssPalmO-2} may be superior to that of LNP_{SM102}.

### (Test Example 7 Evaluation of mRNA Transfection Efficiency after One Intramuscular Immunization)

The mice were intramuscularly immunized with luciferase-derived mRNA-LNPs (1 µg/mouse as the amount of mRNA). D-Luciferin (FUJIFILM Wako Pure Chemical Corporation) was intraperitoneally administered 6 hours, 24 hours, 48 hours, and 72 hours after the immunization, and the luminescence was measured with an in vivo imaging system (Lumina Series III, PerkinElmer Inc.). Also, 6 hours after the immunization, muscles, draining lymph nodes (dLNs), livers, and spleens were collected and homogenized, and luminescence was measured using ONE-Glo EX Luciferase Assay System (Promega Corporation).

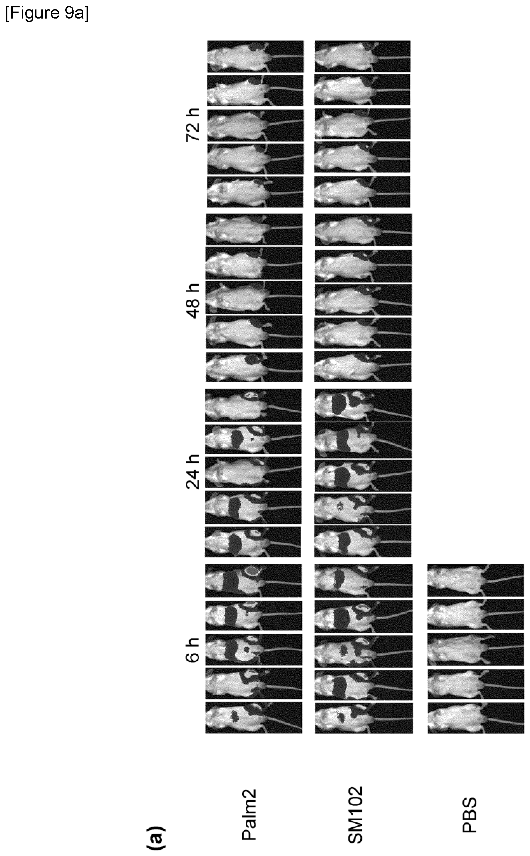

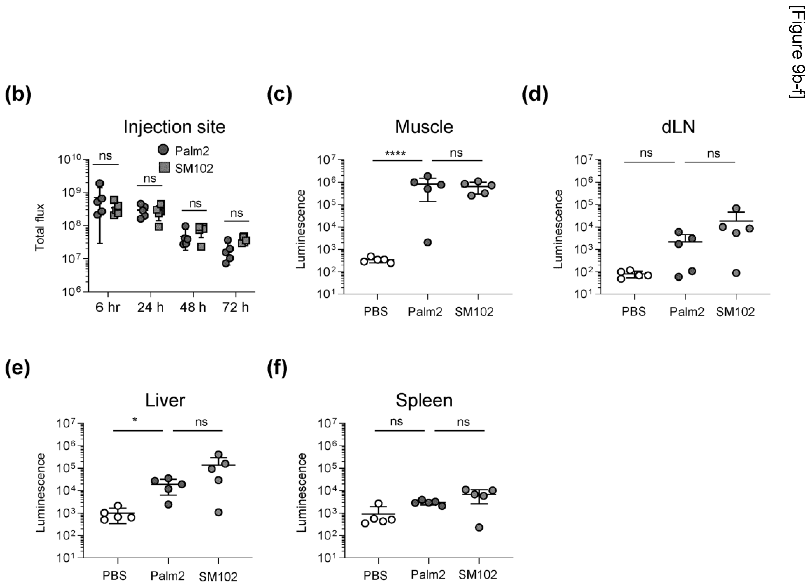

The results of the luminescence measurement are shown in Figure 9. (a) shows the results of observing the change in the luminescence intensity over time in the groups intramuscularly immunized once with the luciferase-derived mRNA using the in vivo imaging system. In the LNP groups, strong fluorescence was observed in the muscles and livers, and the fluorescence gradually disappeared over time. (b) shows the quantified luminescence intensities at the administration sites. Six hours after the administration, luminescence was observed at the administration sites in the LNP groups unlike the PBS group, and no significant difference was observed between the LNPs. The luminescence intensities decreased after 24 hours, but no significant difference was observed between the LNPs at any time point.

(c) to (f) show the results of measuring luciferase activities in the muscles, the draining lymph nodes (dLNs), the livers, and the spleens in the group intramuscularly immunized once with the luciferase-derived mRNA. The luminescence intensities in the muscles and livers significantly increased in the LNP groups compared to the PBS group, but no significant differences were observed between the LNPs.

From the results of the present test example, it was determined that LNP_{ssPalmO-2} showed an mRNA transfection efficiency comparable to that in the LNP_{SM102} group.

### (Test Example 8 Evaluation of mRNA Transfection Efficiency after Two Intramuscular Immunizations)

The mice were intramuscularly immunized with luciferase-derived mRNA-LNPs (1 µg/mouse as the amount of mRNA) on days 0 and 21. D-Luciferin was intraperitoneally administered 6 hours, 24 hours, 48 hours, and 72 hours after the second immunization, and the luminescence was measured with an in vivo imaging system. Also, 6 hours after the second immunization, muscles were collected and homogenized, and luminescence was measured using ONE-Glo EX Luciferase Assay System.

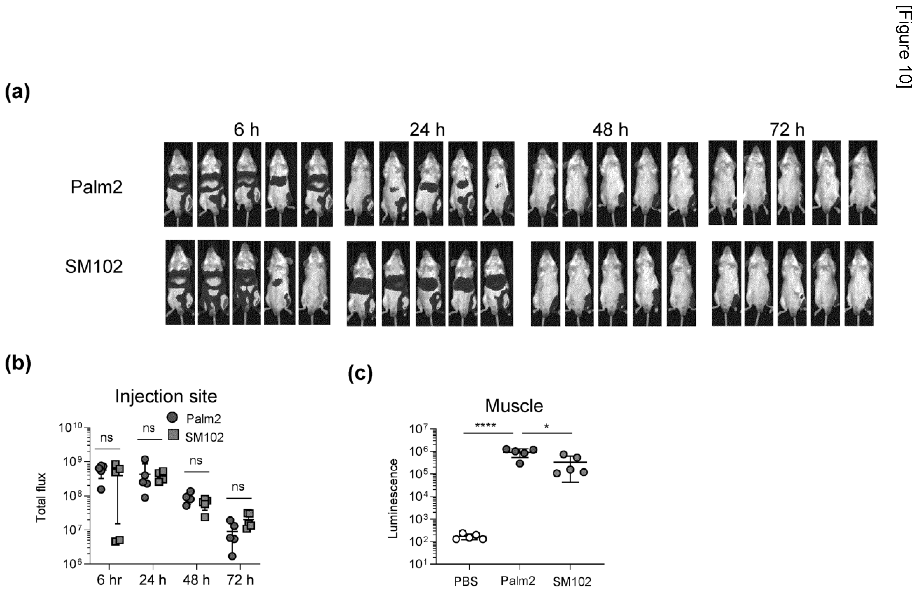

The results of the luminescence measurement are shown in Figure 10. (a) shows the results of observing the change in the luminescence intensity over time using the in vivo imaging system in the groups intramuscularly immunized twice with the luciferase-derived mRNA. In the LNP groups, strong fluorescence was observed in the muscles and livers, and the fluorescence gradually disappeared over time. (b) shows the quantified luminescence intensities at the administration sites. Six hours after the administration, luminescence was observed at the administration sites in the LNP groups, and no significant difference was observed between the LNPs. The luminescence intensities decreased after 24 hours, but no significant difference was observed between the LNPs at any time point.

(c) shows the results of measuring luciferase activities in the muscles in the group intramuscularly immunized twice with the luciferase-derived mRNA. The luminescence intensities in the muscles significantly increased in the LNP groups compared to the PBS group. Although slightly higher, LNP_{ssPalmO-2} showed higher values than LNP_{SM102}.

From the results of the present test example, it was determined that LNP_{ssPalmO-2} exhibited an mRNA transfection efficiency comparable to that exhibited by LNP_{SM102}.

### (Test Example 9 Evaluation of HA Expression after Two Intramuscular Immunizations)

The mice were intramuscularly immunized with HA-derived mRNA-LNPs (1 µg/mouse as the amount of mRNA) on days 0 and 21. Six hours after the second immunization, the muscles were collected, and the HA expression by dendritic cells (DCs) in the muscles was evaluated using a flow cytometer.

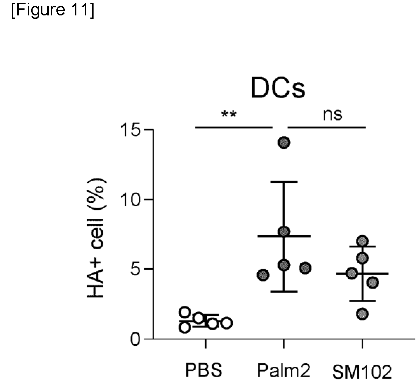

The results of evaluating the HA expressions in the dendritic cells at the administration sites in the groups intramuscularly immunized twice with the HA-derived mRNA are shown in Figure 11. Compared to the PBS group, the proportion of HA-expressing dendritic cells was increased in the LNP groups, and no significant difference was observed between the LNPs. From the results of the present test example, it was determined that LNP_{ssPalmO-2} exhibited an mRNA transfection efficiency comparable to that exhibited by LNP_{SM102}.

### (Test Example 10 Evaluation of Antigen-Presenting Cell Activation in Draining Lymph Nodes after Subcutaneous Immunization)

The mice were subcutaneously immunized with HA-derived mRNA-LNPs (1 µg/mouse as the amount of mRNA), and one day later, the draining lymph nodes were collected. The expression of costimulatory molecules (CD86) on dendritic cells (plasmacytoid DCs, conventional DCs, and migratory DCs), B cells, and macrophages was evaluated using a flow cytometer.

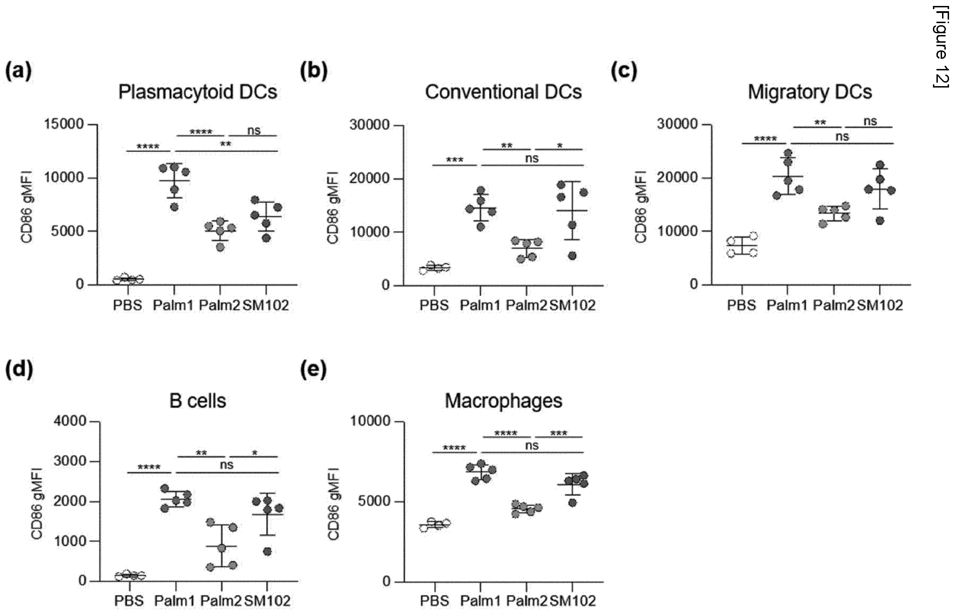

Figure 12 shows the evaluation of antigen-presenting cell activation using CD86 expression as an indicator. The CD86 expression in the dendritic cells, the B cells, and the macrophages was confirmed to be higher with LNP_{SM102} compared to the expression in the non-vaccine group. On the other hand, the CD86 expression tended to be lower with LNP_{ssPalmO-2} than with LNP_{ssPalmO-1} and LNP_{SM102}. The results of the present test example suggested that, while LNP_{ssPalmO-2} induced immune responses (antibody production and T cell response) comparable to those induced by LNP_{ssPalmO-1} and LNP_{SM102}, the innate immune activation abilities of the LNPs were low.

### (Test Example 11 Evaluation of Blood Cytokine Levels after Subcutaneous Immunizations)

The mice were subcutaneously immunized with HA-derived mRNA-LNPs (1 µg/mouse as the amount of mRNA) on days 0 and 21. On days 0 and 21, blood was collected 6 hours after the subcutaneous immunizations, and blood cytokine levels were measured using LEGENDplex (BioLegend, Inc.).

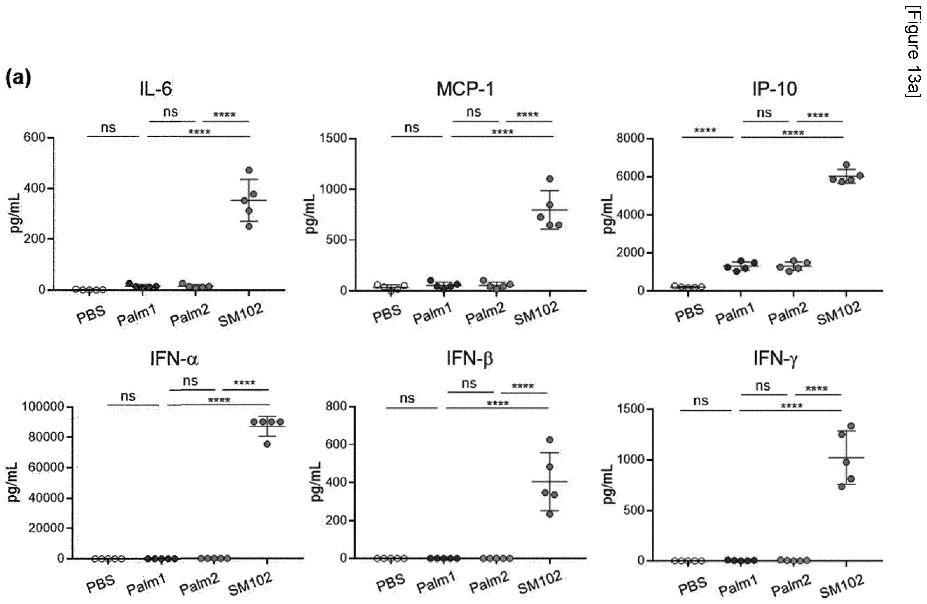

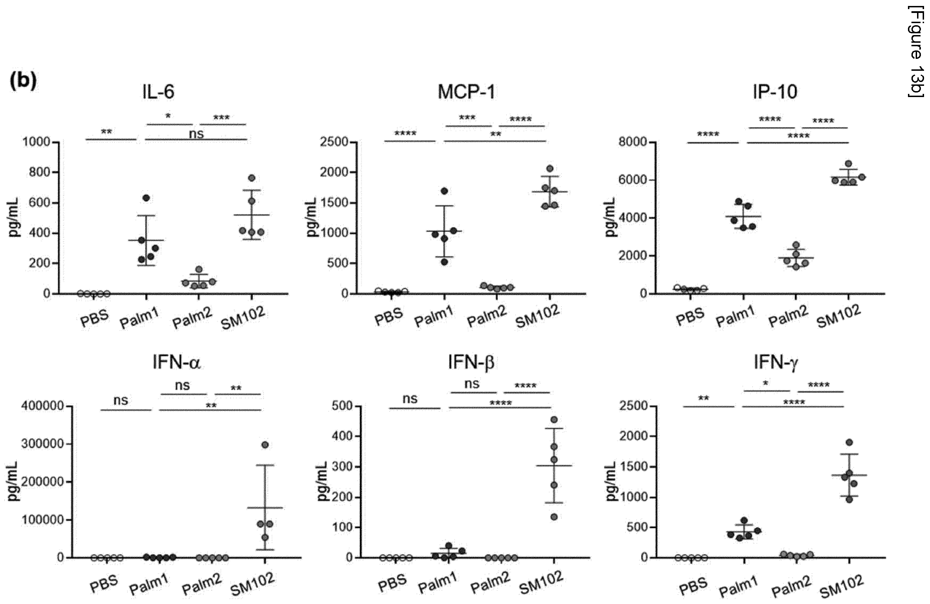

Figure 13 shows the blood cytokine levels 6 hours after (a) the first subcutaneous immunization (Prime) and (b) the second subcutaneous immunization (Boost). LNP_{ssPalmO-2} showed significantly lower levels of the inflammatory cytokines or chemokines IFN-α, IFN-β, IFN-γ, MCP-1, IP-10, and IL-6 compared to LNP_{SM102}. Although not as low as those shown with LNP_{ssPalmO-2}, the levels were also found to be significantly lower with LNP_{ssPalmO-1} compared to LNP_{SM102}. From the results of the present test example, it was shown that LNP_{ssPalmO-2} may induce the vaccine effect comparable to that induced by LNP_{SM102} while reducing adverse reactions.

### (Test Example 12 Evaluation of Blood Cytokine Levels after Subcutaneous Immunizations)

Measurements were performed in the same manner as in Test Example 11, except that the levels of cytokines and chemokines different from those in Test Example 11 were measured.

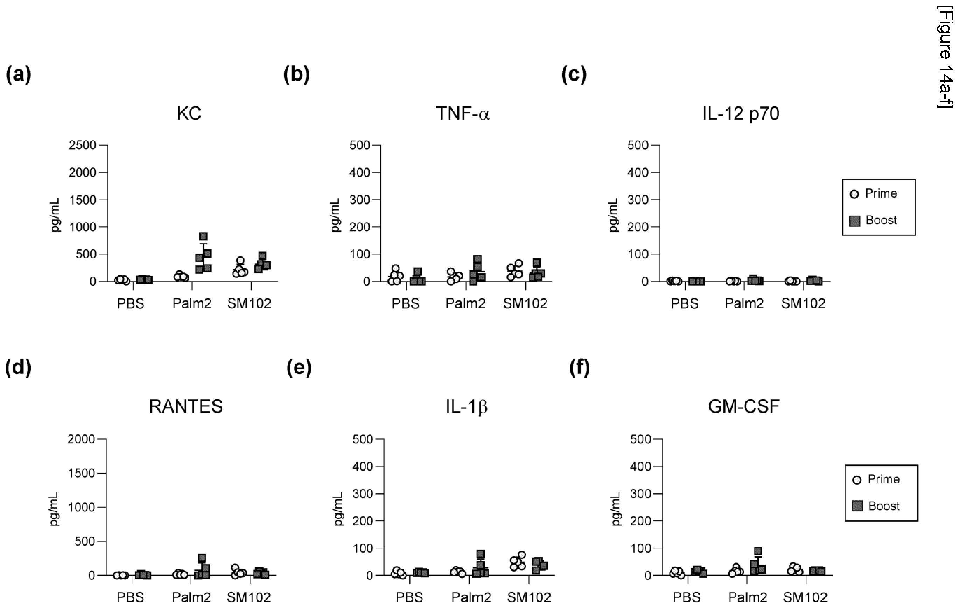

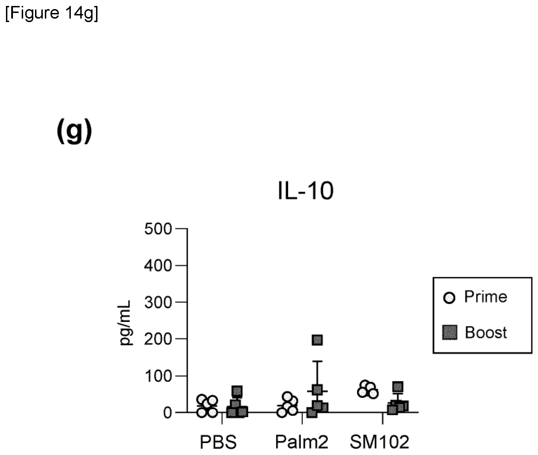

Figure 14 shows the blood cytokine levels 6 hours after the first subcutaneous immunization (Prime) and the second subcutaneous immunization (Boost). The inflammatory cytokines or chemokines KC, TNF-α, IL-12 p70, RANTES, IL-1β, GM-CSF, and IL-10 were hardly induced by any of the LNPs.

### (Test Example 13 Evaluation of Blood Cytokine Levels after Intramuscular Immunizations)

The mice were intramuscularly immunized with HA-derived mRNA-LNPs (1 µg/mouse as the amount of mRNA) on days 0 and 21. On days 0 and 21, blood was collected 6 hours after the intramuscular immunizations, and blood cytokine levels were measured using LEGENDplex.

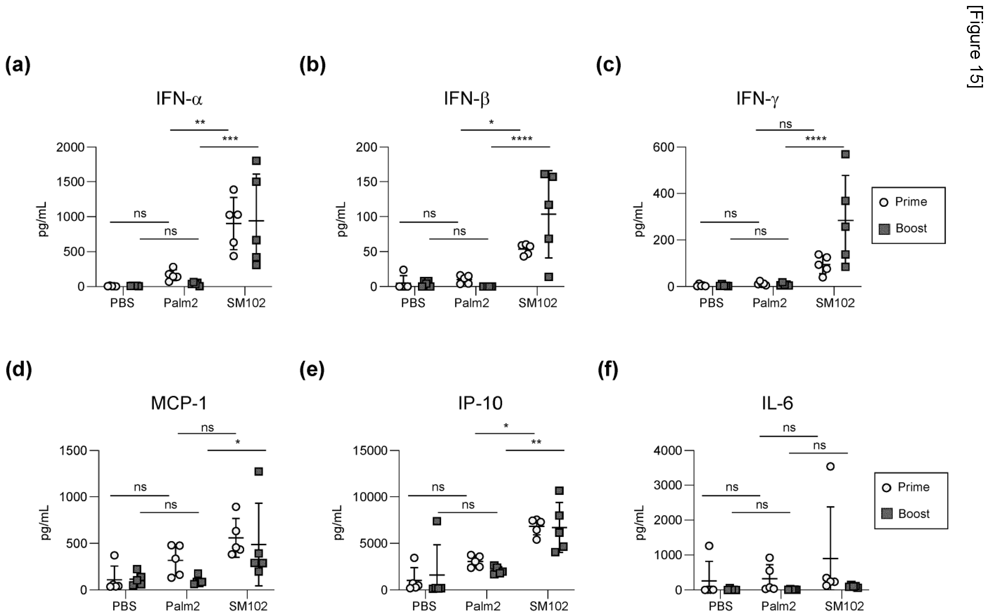

Figure 15 shows the blood cytokine levels 6 hours after the first intramuscular immunization (Prime) and the second intramuscular immunization (Boost). LNP_{ssPalmO-2} showed significantly lower levels of the inflammatory cytokines or chemokines IFN-α, IFN-β, IFN-γ, MCP-1, and IP-10 compared to LNP_{SM102}. From the results of the present test example, it was shown that LNP_{ssPalmO-2} may induce the vaccine effect comparable to that induced by LNP_{SM102} while reducing adverse reactions, even when administered by intramuscular administration.

### (Test Example 14 Evaluation of Adverse Reactions after Intramuscular Immunization)

After the mice were intramuscularly immunized with HA-derived mRNA-LNPs (1 µg/mouse as the amount of mRNA), Evans blue was administered intravenously. Six hours later, the muscles were collected and homogenized, and the amounts of Evans blue in the muscles was measured to evaluate vascular permeability at the administration site, that is, inflammation at the administration site.

When measuring the body weight as an indicator of an adverse reaction, the body weight was measured 24 hours after the intramuscular immunization with the HA-derived mRNA-LNPs (20 µg/mouse as the amount of mRNA).

When measuring the body temperature as an indicator of an adverse reaction, the mice were intramuscularly immunized with the HA-derived mRNA-LNPs (5 µg/mouse as the amount of mRNA) on days 0 and 21. On days 0 and 21, the body temperature was measured 6 hours after the intramuscular immunization.

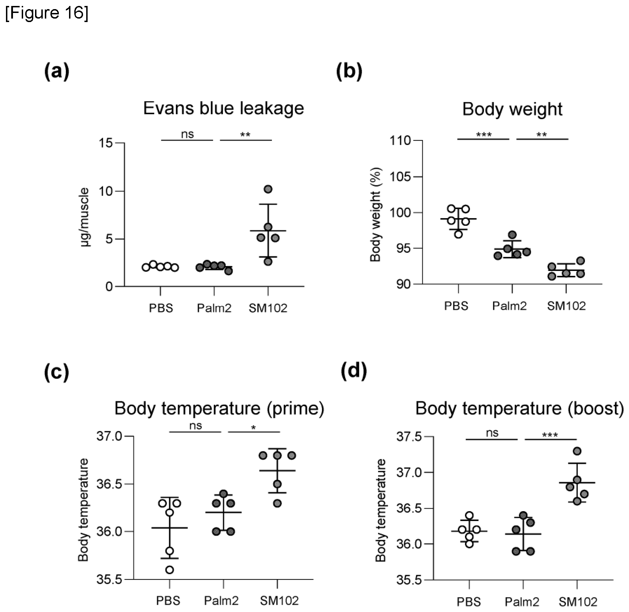

The evaluation of the adverse reaction after the intramuscular immunization is shown in Figure 16. (a) shows the amounts of Evans blue in the muscles 6 hours after the intramuscular immunization. The amount of Evans blue at the administration site was shown to be significantly lower in the LNP_{ssPalmO-2} group compared to the LNP_{SM102} group, indicating that inflammation, which was an adverse reaction, was reduced.

(b) shows the changes in the body weight 24 hours after the intramuscular immunization. Although all types of LNPs induced a body weight loss, the LNP_{ssPalmO-2} group showed significantly less body weight loss than the LNP_{SM102} group.

(c) and (d) show the body temperatures 6 hours after the first intramuscular immunization (Prime) and the second intramuscular immunization (Boost). The rise in body temperature was shown to be significantly reduced in the LNP_{ssPalmO-2} group compared to the LNP_{SM102} group.

From the results of the present test example, LNP_{ssPalmO-2} was shown to cause less adverse reactions than LNP_{SM102}.

### (Test Example 15 Comparison of Neutral Lipids)

DSPC-comprising LNP_{ssPalmO-2} was prepared in the same manner as LNP_{ssPalmO-2}, except that DSPC was used instead of DOPC. In the figures described below, DOPC-comprising LNP_{ssPalmO-2}, DSPC-comprising LNP_{ssPalmO-2}, and DSPC-comprising LNP_{SM102} are referred to as Palm2 (DOPC), Palm2 (DSPC), and SM102(DSPC), respectively.

The mice were subcutaneously immunized with HA-derived mRNA-LNPs (1 µg/mouse as the amount of mRNA) on days 0 and 21. Blood was collected 6 hours after the second immunization, and cytokine levels were measured using LEGENDplex. On day 35, blood was collected, and rHA-specific IgG1, IgG2b, and IgG2c in the plasma were evaluated by ELISA.

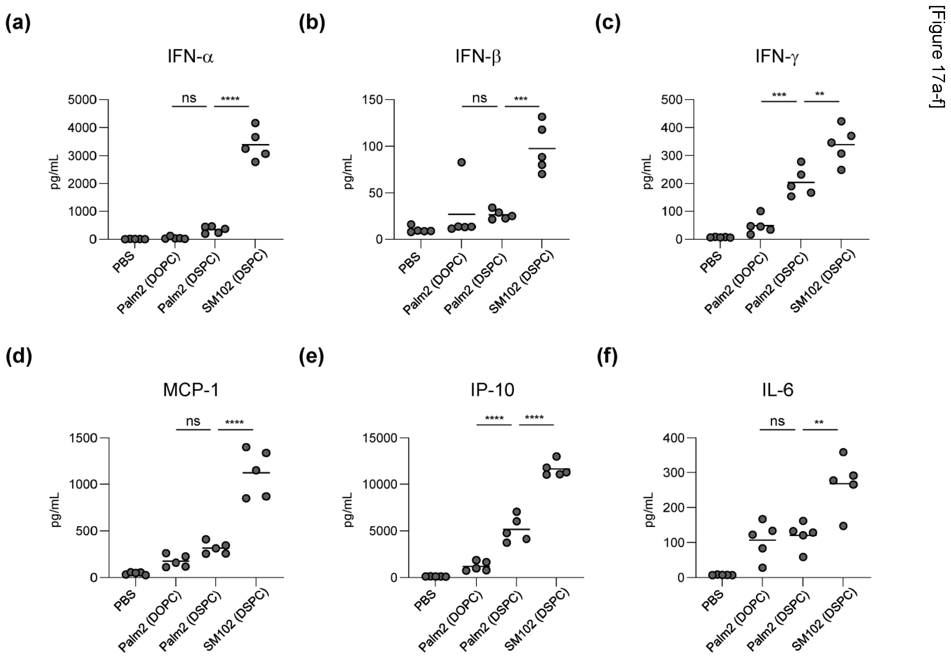

The blood cytokine levels are shown in Figure 17(a) to (f). DSPC-comprising LNP_{ssPalmO-2} showed significantly increased levels of the inflammatory cytokines or chemokines IFN-γ and IP-10 compared to DOPC-comprising LNP_{ssPalmO-2}. Also, DSPC-comprising LNP_{ssPalmO-2} showed significantly lower levels of IFN-α, IFN-γ, IFN-γ, IL-6, MCP-1, and IP-10 compared to DSPC-comprising LNP_{SM102}.

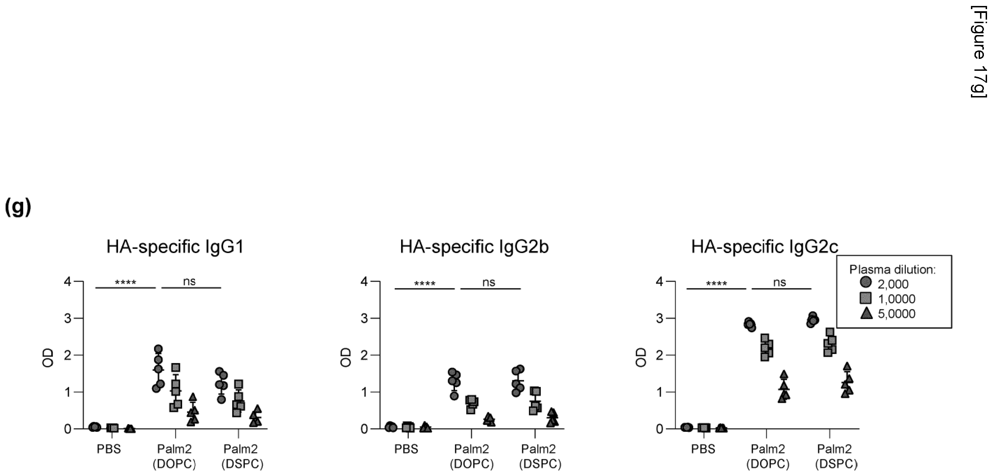

The rHA-specific IgGs in the plasma are shown in Figure 17(g). Higher antibody titers were observed in the mRNA-LNP group compared to the non-vaccine group. No significant differences were observed between the LNPs.

From the results of the present test example, it was shown that DOPC-comprising LNP_{ssPalmO-2} may induce the vaccine effect comparable to that induced by DSPC-comprising LNP_{ssPalmO-2} while reducing adverse reactions. On the other hand, the prophlogistic properties of DSPC-comprising LNP_{ssPalmO-2} was significantly reduced compared to DSPC-comprising LNP_{SM102}, suggesting that the main cause of the reduction in the prophlogistic properties of LNP_{ssPalmO-2} was the use of ssPalmO.

### (Test Example 16 Evaluation of Blood Cytokine Levels after Intramuscular Immunizations)

mRNA-LNPs were obtained according to the procedure described above under "Experimental Animals, Reagents, and the like", except that the mRNA was changed as follows.
- mRNA: Spike protein derived from a novel coronavirus (SARS-CoV-2: origin strain and variant). Uridine in the mRNA is replaced with N1-methylpseudouridine.

Mice are intramuscularly immunized with SARS-CoV-2-derived mRNA-LNPs. Blood is collected 6 hours after the intramuscular immunization, and blood cytokine levels are measured using LEGENDplex.

It was shown that, compared to LNP_{SM102}, LNP_{ssPalmO-2} can significantly reduce the inflammatory cytokine or chemokine levels, thus reducing adverse reactions.

### Summary

It was found that the LNPs prepared using ssPalmO as the ionic lipid (LNP_{ssPalmO}) induced the vaccine effect comparable to that of commonly used LNPs, while being less prophlogistic. Specifically, using hemagglutinin (HA) and neuraminidase (NA) mRNAs derived from the highly pathogenic avian influenza virus subtype H5N1 (H5N1 virus) as model antigens, the vaccine effects and prophlogistic properties of LNPs (LNP_{SM102}) which were prepared using SM102, an ionic lipid commonly used worldwide, and LNP_{ssPalmO} were evaluated in mice. As a result, LNP_{ssPalmO} induced antigen-specific antibody production and T cell responses that were comparable to or greater than those induced by LNP_{SM102}. Furthermore, when the mice were infected after the vaccination with an H5N1 virus of the same strain as the vaccine strain, significant body weight loss was observed, and all mice died within 14 days in the non-vaccine group. On the other hand, in the LNP_{ssPalmO} and LNP_{SM102} vaccine groups, no weight loss was observed, and all mice survived. When the mice were infected after the vaccination with an H5N1 virus strain that was different from the vaccine strain, the body weight loss was improved with LNP_{ssPalmO} compared to LNP_{SM102}. Moreover, compared to LNP_{SM102}, LNP_{ssPalmO} was found to significantly and markedly reduce the post-vaccination production of inflammatory cytokines and chemokines such as IL-6, IFN-γ, and MCP-1, which were the indicators of prophlogistic properties that were the main cause of the adverse reactions. The above results demonstrated that LNP_{ssPalmO} is a novel LNP capable of inducing the vaccine effect and the ability to protect against an infection comparable to those induced by LNP_{SM102}, while reducing adverse reactions.

## Claims

1. A composition for use in a vaccine, the composition comprising:
a cationic lipid represented by the following formula (1): 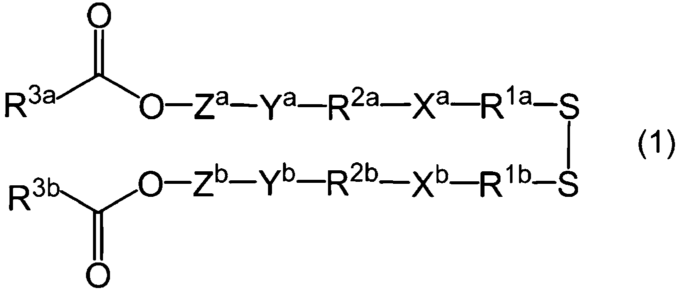
(In Formula (1),
R^{1a} and R^{1b} each independently represent an alkylene group having 1 to 6 carbon atoms,
X^{a} and X^{b} each independently represent an acyclic alkyl tertiary amino group having one tertiary amino group wherein the alkyl has 1 to 6 carbon atoms, or a cyclic alkylene tertiary amino group having one or two tertiary amino groups wherein the alkylene has 2 to 5 carbon atoms,
R^{2a} and R^{2b} each independently represent an alkylene or an oxydialkylene group having 8 or less carbon atoms,
Y^{a} and Y^{b} each independently represent an ester bond, an amide bond, a carbamate bond, an ether bond, or a urea bond,
Z^{a} and Z^{b} each independently represent a divalent group derived from an aromatic compound, the aromatic compound having 3 to 16 carbon atoms and at least one aromatic ring and optionally having a heteroatom, and
R^{3a} and R^{3b} each independently represent a residue derived from a reaction product of a fat-soluble vitamin having a hydroxyl group and succinic anhydride or glutaric anhydride, a residue derived from a reaction product of a sterol derivative having a hydroxyl group and succinic anhydride or glutaric anhydride, or an aliphatic hydrocarbon group having 12 to 22 carbon atoms).

2. The composition according to claim 1, further comprising a nucleic acid.

3. The composition according to claim 2, wherein
a ratio of a content of the cationic lipid and a content of the nucleic acid, as a content of cationic lipid (nmol)/content of nucleic acid (µg), is 0.05 to 150.

4. The composition according to any of claims 1 to 3, further comprising one or more selected from the group consisting of an ionizable lipid, a neutral lipid, a PEG-modified lipid, and a sterol.

5. The composition according to claim 4, which is lipid particles.

6. The composition according to claim 2 or 3, which is for preventing or treating an infectious disease.

7. The composition according to claim 2, which is for intradermal administration, subcutaneous administration, transnasal administration, or intramuscular administration.
